(19) Europäisches Patentamt
European Patent Office
Office européen des brevets

(11) **EP 2 155 058 B1**

(12) **EUROPEAN PATENT SPECIFICATION**

(45) Date of publication and mention
of the grant of the patent:
**16.09.2015 Bulletin 2015/38**

(51) Int Cl.:
*A61B 5/00* *(2006.01)* *A61B 5/053* *(2006.01)*

(21) Application number: **08747944.0**

(22) Date of filing: **13.05.2008**

(86) International application number:
**PCT/AU2008/000673**

(87) International publication number:
**WO 2008/138062 (20.11.2008 Gazette 2008/47)**

(54) **INDICATOR**

INDIKATOR

INDICATEUR

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **14.05.2007 AU 2007902540 P**

(43) Date of publication of application:
**24.02.2010 Bulletin 2010/08**

(73) Proprietor: **Impedimed Limited
Pinkenba, QLD 4008 (AU)**

(72) Inventors:
• **CHETHAM, Scott
Cardiff by the Sea, California 92007 (US)**

• **ESSEX, Tim
Clayfield, Queensland 4011 (AU)**
• **ZIEGELAAR, Brian, William
Brisbane, Queensland 4154 (AU)**

(74) Representative: **Gray, Peter John Bracey
Thompson Gray LLP
Central Court
25 Southampton Buildings
London WC2A 1AL (GB)**

(56) References cited:
**EP-A1- 1 629 772**    **WO-A1-2007/041783**
**WO-A1-2007/041783**    **US-A1- 2004 167 423**
**US-A1- 2004 167 423**    **US-A1- 2006 224 079**
**US-A1- 2006 224 079**    **US-B2- 6 714 813**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Background of the Invention

The present invention relates to a method and apparatus for use in analysing impedance measurements performed on a subject, and in particular to a method and apparatus for displaying an indicator that is indicative of a subject parameter, such as fluid levels within the subject.

### Description of the Prior Art

The reference in this specification to any prior publication (or information derived from it), or to any matter which is known, is not, and should not be taken as an acknowledgment or admission or any form of suggestion that the prior publication (or information derived from it) or known matter forms part of the common general knowledge in the field of endeavour to which this specification relates.

One existing technique for determining biological parameters relating to a subject, such as fluid levels, involves the use of bioelectrical impedance. This involves measuring the electrical impedance of a subject's body using a series of electrodes placed on the skin surface. Changes in electrical impedance at the body's surface are used to determine parameters, such as changes in fluid levels, associated with the cardiac cycle or oedema, or other conditions which affect body habitus.

Lymphoedema is a condition characterised by excess protein and oedema in the tissues as a result of reduced lymphatic transport capacity and/or reduced tissue proteolytic capacity in the presence of a normal lymphatic load. Acquired, or secondary lymphoedema, is caused by damaged or blocked lymphatic vessels. The commonest inciting events are surgery and/or radiotherapy. However, onset of lymphoedema is unpredictable and may develop within days of its cause or at any time during a period of many years after that cause.

WO00/79255 describes a method of detection of oedema by measuring bioelectrical impedance at two different anatomical regions in the same subject at a single low frequency alternating current. The two measurements are analysed to obtain an indication of the presence of tissue oedema by comparing with data obtained from a normal population.

WO 2007/041783 describes a method of determining an indication of the hydration status of a subject. An impedance value is measured for at least one body segment, and the measured impedance value is used to determine at least one indicator at least partially indicative of a level of extracellular fluid. Indicators can then be used to determine an indication of the hydration status of a subject.

US2004/0167423 relates to methods and systems for displaying biometric impedance values as vectors on a graph for diagnostic and prognostic analysis of patients and patient populations. The graph may be based on a whole body bioelectrical impedance analysis, or a partial body bioelectrical impedance analysis.

US 2006/0224079 relates to a system and a method for edema monitoring using an implantable medical device. The inter-thoracic impedance of a patient is monitored by an implantable medical device. The implantable medical device transmits the measured impedance to a personal monitor to provide a representation related to the measured impedance to the patient.

In view of the different types of impedance measurement that can be performed, operators of impedance monitoring units have to be knowledgeable regarding their operation. In some situations, machines are only adapted to provide only one form of impedance analysis and as a result, provide a standard output that must then be interpreted by the operator. However, in the event that different operating modes can be selected, it is necessary for the operator of the machine to be aware of any intricacies associated with the selected measurement mode, as well as being able to interpret the different outputs that may be available.

Summary of the Present Invention

It is an object of the present invention to substantially overcome, or at least ameliorate, one or more disadvantages of existing arrangements.

In a first broad form the present invention provides a method for use in determining an oedema indicator, the method including, in a processing system:

    a) determining at least one impedance value, representing the impedance of at least a segment of the subject, the at least one impedance value including:

        i) a first measured impedance value representing the impedance of an unaffected limb;
        ii) a second measured impedance value representing the impedance of an affected limb;

b) determining an indicator indicative of a subject parameter using the at least one impedance value and a reference, wherein the indicator is the oedema indicator and is scaled relative to at least one reference value by:

 i) determining an impedance ratio using the first and second measured impedance values by;

  a. determining a mean impedance ratio value for a normal population;
  b. determining a three standard deviation value for the normal population; and,

 ii) determining the oedema indicator by scaling the impedance ratio using the mean and three standard deviation values using the equation:

$$L - Dex = \frac{sf \times (IR - \mu)}{3\sigma - \mu}$$

 where:

  L-Dex is the indicator
  IR is the impedance ratio
  $\mu$ is the mean impedance ratio value for a normal population
  $3\sigma$ is the three standard deviation value for the normal population
  sf is the scaling factor; and,

c) displaying a representation of the oedema indicator.

Typically the subject parameter is at least one of:

a) fluid levels within the subject; and,
b) extracellular fluid levels in a subject limb.

Typically the method includes, in the processing system, determining the impedance ratio using the equation:

$$IR = \frac{Zul}{Zal}$$

where:

Zul is the measured impedance of the unaffected limb
Zal is the measured impedance of the affected limb

Typically the method includes, in the processing system:

a) determining one or more impedance parameter values from measured impedance values, the impedance parameter values include at least one of:

 i) an impedance at infinite applied frequency ($R_\infty$);
 ii) an impedance at zero applied frequency ($R_0$); and,
 iii) an impedance at a characteristic frequency (Zc); and,

b) determining the impedance ratio using the impedance parameter values.

Typically the method includes, in the processing system, determining the impedance ratio using the equation:

$$IR = \frac{R_0 ul}{R_0 al}$$

where:

IR is the impedance ratio
$R_0 ul$ is the impedance of the unaffected limb at zero frequency
$R_0 al$ is the impedance of the affected limb at zero frequency

Typically the scaling factor is at least one of:

a) an integer value;
b) a multiple of ten.

Typically the reference is at least one of:

a) derived from a normal population; and,
b) determined from predetermined values.

Typically the method includes, in the processing system:

a) determining one or more subject details, the subject details include at least one of:

i) limb dominance;
ii) ethnicity;
iii) age;
iv) sex;
v) weight; and,
vi) height; and,

b) selecting the reference at least partially in accordance with the subject details.

Typically the method includes, in the processing system, displaying the representation as a linear scale, the linear scale including:

a) a linear indicator;
b) a scale; and,
c) a pointer, the pointer being positioned on the scale in accordance with the indicator.

Typically the method includes, in the processing system, displaying the representation as a linear scale, the linear scale including:

a) a linear indicator;
b) a scale;
c) at least one bar representing the indicator value; and,
d) at least one bar representing at least one of a previous indicator value and a baseline indicator value.

Typically the method includes, in the processing system:

a) determining at least one threshold using the reference; and,
b) displaying the threshold on the representation, the position of the threshold being indicative of the presence of a condition.

Typically the method includes, in the processing system, displaying, on the representation, at least one of:

a) a normal range;

b) an intervention range;

c) a hydration range;

d) an oedema range;

e) at least one colour coded region representing a respective range; and,

f) a Gaussian curve representing a normal population distribution.

Typically the method includes, in the processing system causing one or more impedance measurements to be performed by:

a) causing at least one excitation signals to be applied to the subject;

b) determining an at least one signal measured across the subject; and,

c) determining at least one impedance value using an indication of the excitation signal and the signal measured across the subject.

In a second broad form the present invention provides apparatus for use in determining an oedema indicator, the apparatus including a processing system for:

a) determining at least one impedance value, representing the impedance of at least a segment of the subject, the at least one impedance value including:

i) a first measured impedance value representing the impedance of an unaffected limb;

ii) a second measured impedance value representing the impedance of an affected limb;

b) determining an indicator indicative of a subject parameter using the at least one impedance value and a reference, wherein the indicator is the oedema indicator and is scaled relative to at least one reference value by:

i) determining an impedance ratio using the first and second measured impedance values by;

a. determining a mean impedance ratio value for a normal population;

b. determining a three standard deviation value for the normal population; and,

ii) determining the oedema indicator by scaling the impedance ratio using the mean and three standard deviation values using the equation:

$$L - Dex = \frac{sf \times (IR - \mu)}{3\sigma - \mu}$$

where:

L-Dex is the indicator

IR is the impedance ratio

$\mu$ is the mean impedance ratio value for a normal population

$3\sigma$ is the three standard deviation value for the normal population

$sf$ is the scaling factor; and,

c) displaying a representation of the oedema indicator.

Typically the apparatus includes:

a) a signal generator for applying one or more electrical signals to the subject using a first set of electrodes;

b) a sensor for measuring electrical signals across a second set of electrodes applied to the subject; and,

c) a controller for:

i) controlling the signal generator; and,

ii) determining the indication of the measured electrical signals.

It will be appreciated that the broad forms of the invention may be used individually or in combination, and may be used for diagnosis of the presence, absence or degree of a range of conditions and illnesses, including, but not limited to oedema, lymphoedema, body composition and the like.

Brief Description of the Drawings

An example of the present invention will now be described with reference to the accompanying drawings, in which: -

Figure 1 is a schematic of an example of impedance determination apparatus;

Figure 2 is a flowchart of an example of a process for determining an indicator;

Figures 3A to 3C are schematic diagrams of first examples of representations of oedema indicators;

Figure 4 is a flowchart of an example of a process for determining an oedema indicator for uni-lateral limb oedema;

Figures 5A and 5B are diagrams of examples of electrode positions for use in measuring limb impedances;

Figures 5C and 5D are schematic diagrams of examples of electrode positions for use in measuring limb impedances;

Figures 6A to 6D are schematic diagrams of second examples of representations of oedema indicators, showing additional information;

Figures 7A and 7B are schematic diagrams of third examples of representations of oedema indicators, showing variations in the oedema indicator from a baseline;

Figures 8A to 8C are schematic diagrams of further examples of representations showing historical variations in oedema indicators;

Figure 9 is a schematic representation of an example of a report incorporating representation of oedema indicators;

Figure 10A is a flow chart of an example of a process for determining hydration and other body composition indicators;

Figure 10B is a schematic diagram of an example of a compartment model for body composition;

Figure 10C is a schematic diagram of an example of electrode positions for use in measuring whole body impedance;

Figure 10D is an example of a Piccoli plot for use in determining a hydration index;

Figure 11A is a schematic representation of an example of a report incorporating hydration indicators;

Figures 11B and 11C are schematic representations of examples of hydration indicators;

Figure 12A is a schematic representation of an example of a report incorporating fat mass indicators;

Figure 12B is a schematic representations of an example of a fat mass indicator;

Figure 13 is a schematic representation of an example of a body composition report; and,

Figure 14 is a schematic representation of an example of a active tissue mass report.

**Detailed Description of the Preferred Embodiments**

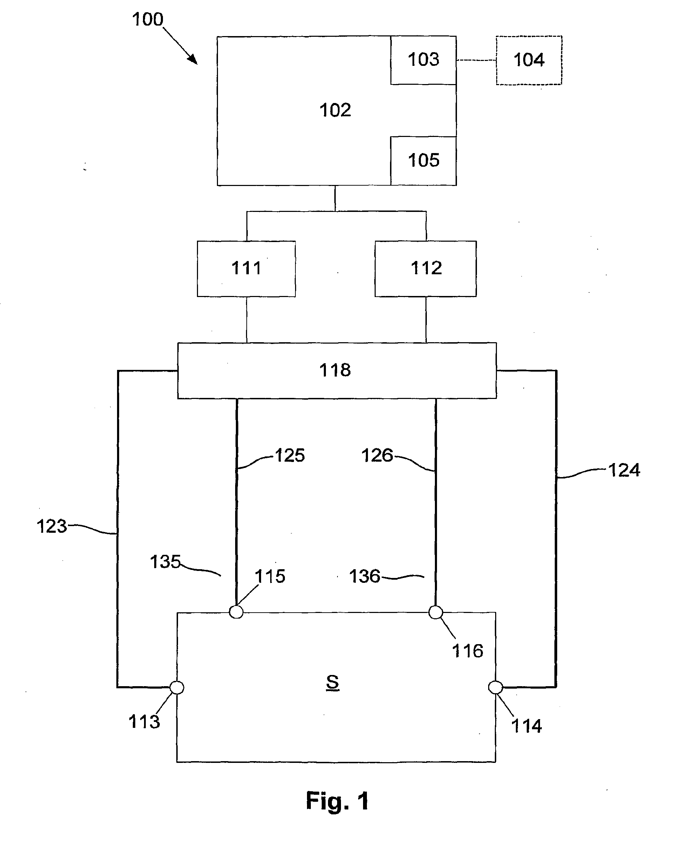

An example of apparatus suitable for performing an analysis of a subject's bioelectric impedance will now be described with reference to Figure 1.
As shown the apparatus includes a measuring device 100 including a processing system 102 coupled to a signal generator 111 and a sensor 112. In use the signal generator 111 and the sensor 112 are coupled to first electrodes 113, 114, and second electrodes 115, 116, provided on a subject S, via respective first leads 123, 124, and second leads 125, 126. The connection may be via a switching device 118, such as a multiplexer, allowing the leads 123, 124, 125, 126 to be selectively interconnected to signal generator 111 and the sensor 112, although this is not essential, and connections may be made directly between the signal generator 111 and the first electrodes 113, 114, and the sensor 112 and the second electrodes 115, 116.

An optional external interface 103 can be used to couple the measuring device 100, via wired, wireless or network connections, to one or more peripheral devices 104, such as an external database or computer system, barcode scanner, or the like. The processing system 102 will also typically include an I/O device 105, which may be of any suitable form such as a touch screen, a keypad and display, or the like.

In use, the processing system 102 is adapted to generate control signals, which causes the signal generator 111 to generate one or more alternating signals, such as voltage or current signals, which can be applied to a subject S, via the first electrodes 113, 114. The sensor 112 then determines the voltage across or current through the subject S, using the second electrodes 115, 116 and transfers appropriate signals to the processing system 102.

Accordingly, it will be appreciated that the processing system 102 may be any form of processing system which is suitable for generating appropriate control signals and interpreting an indication of the measured signals to thereby determine the subject's bioelectrical impedance, and optionally determine other information such as the presence, absence or degree of oedema, or the like.

The processing system 102 may therefore be a suitably programmed computer system, such as a laptop, desktop, PDA, smart phone or the like. Alternatively the processing system 102 may be formed from specialised hardware, such as an FPGA (field programmable gate array), or a combination of a programmed computer system and specialised hardware, or the like.

It will be appreciated that the processing system 102, the signal generator 111 and the sensor 112 may be integrated into a common housing and therefore form an integrated device. Alternatively, the processing system 102 may be connected to the signal generator 111 and the sensor 112 via wired or wireless connections. This allows the processing system 102 to be provided remotely to the signal generator 111 and the sensor 112. Thus, the signal generator 111 and the sensor 112 may be provided in a unit near, or worn by the subject S, whilst the processing system 102 is situated remotely to the subject S.

In use, the first electrodes 113, 114 are positioned on the subject to allow one or more signals to be injected into the subject S. The location of the first electrodes will depend on the segment of the subject S under study, and can include for example, positioning electrodes on the wrist and ankles of a subject, to allow the impedance of limbs, or the whole body, to be determined.

Once the electrodes are positioned, one or more alternating signals are applied to the subject S, via the first leads 123, 124 and the first electrodes 113, 114. The nature of the alternating signal will vary depending on the nature of the measuring device and the subsequent analysis being performed.

For example, the system can use Bioimpedance Analysis (BIA) in which a single low frequency current is injected into the subject S, with the measured impedance being used directly in the assessment of oedema. In contrast Bioimpedance Spectroscopy (BIS) devices utilise frequencies ranging from very low frequencies (4 kHz) to higher frequencies (1000 kHz), and can use 256 or more different frequencies within this range, to allow multiple impedance measurements to be made within this range.

Thus, the measuring device 100 may either apply an alternating signal at a single frequency, at a plurality of frequencies simultaneously, or by apply a number of alternating signals at different frequencies sequentially, depending on the preferred implementation. The frequency or frequency range of the applied signals may also depend on the analysis being performed.

In one example, the applied signal is a frequency rich current from a current source clamped, or otherwise limited, so it does not exceed a maximum allowable subject auxiliary current. However, alternatively, voltage signals may be applied, with a current induced in the subject being measured. The signal can either be constant current, impulse function or a constant voltage signal where the current is measured so it does not exceed the maximum allowable subject auxiliary current.

A potential difference and/or current are measured between the second electrodes 115, 116. The acquired signal and the measured signal will be a superposition of potentials generated by the human body, such as the ECG, and potentials generated by the applied current.

Optionally the distance between the second electrodes may be measured and recorded. Similarly, other parameters relating to the subject may be recorded, such as the height, weight, age, sex, health status, any interventions and the date and time on which they occurred. Other information, such as current medication, may also be recorded.

To assist accurate measurement of the impedance, buffer circuits may be placed in connectors that are used to connect the second electrodes 115, 116 to the second leads 125, 126. This ensures accurate sensing of the voltage response of the subject S, and in particular helps eliminate contributions to the measured voltage due to the response of the second leads 125, 126, and reduce signal loss.

This in turn greatly reduces artefacts caused by movement of the second leads 125, 126, which is particularly important in some applications such as monitoring fluid levels during dialysis, in which sessions usually last for several hours and the subject will move around and change positions during this time.

A further option is for the voltage to be measured differentially, meaning that the sensor used to measure the potential at each second electrode 115, 116 only needs to measure half of the potential as compared to a single ended system.

The measurement system may also have buffers placed in the connectors between the first electrodes 113, 114 and the first leads 123, 124. In one example, current can also be driven or sourced through the subject S differentially, which again greatly reduced the parasitic capacitances by halving the common-mode current. Another particular advantage of using a differential system is that the micro-electronics built into the connectors for each first electrode 113, 114 also removes parasitic capacitances that arise when the subject S, and hence the leads first 123, 124, move.

The acquired signal is demodulated to obtain the impedance of the system at the applied frequencies. One suitable method for demodulation of superposed frequencies is to use a Fast Fourier Transform (FFT) algorithm to transform the time domain data to the frequency domain. This is typically used when the applied current signal is a superposition of applied frequencies. Another technique not requiring windowing of the measured signal is a sliding window FFT.

In the event that the applied current signals are formed from a sweep of different frequencies, then it is more typical to use a processing technique such as multiplying the measured signal with a reference sine wave and cosine wave derived from the signal generator, or with measured sine and cosine waves, and integrating over a whole number of cycles. This process rejects any harmonic responses and significantly reduces random noise.

Other suitable digital and analog demodulation techniques will be known to persons skilled in the field.

In the case of BIS, impedance or admittance measurements are determined from the signals at each frequency by comparing the recorded voltage and current signal. The demodulation algorithm will produce an amplitude and phase signal at each frequency.

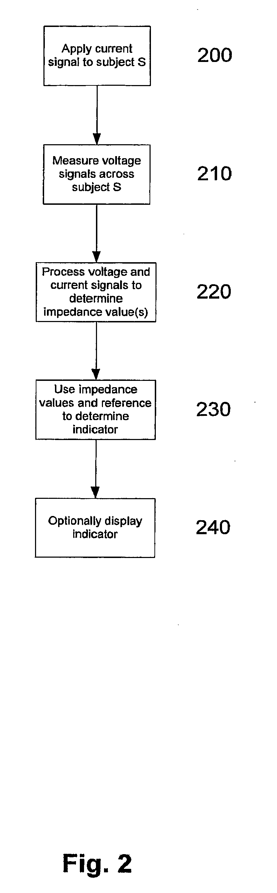

An example of the operation of the apparatus to generate an oedema indicator will now be described with reference to Figure 2.

In this example, at step 200 the processing system 102 causes a current signal to be applied to the subject S, with the induced voltage across the subject S being measured at step 210, with signals representing the measured voltage and the applied current being returned to the processing system 102 for analysis.

When the process is being used to determine an oedema indicator, this is typically performed for at least a segment of the subject S that is suspected of being susceptible to oedema, and may also be repeated for a separate healthy segment of the subject. Thus, for example, in the case of limb oedema, this is typically performed on the affected or "at risk" limb (hereinafter generally referred to as the "affected" limb), and the contra-lateral limb. However, if the process is being used to determine other indicators, such as hydration indicators, then whole body impedance measurements may be performed, in which case the segment corresponds to the entire subject.

It will be appreciated that the application of the current and voltage signals may be controlled by a separate processing system to that used in performing the analysis to derive an indicator, and that the use of a single processing system is for the purpose of example only.

At step 220, measured voltage and current signals are used by the processing system 102 to determine one or more measured impedance values, depending on whether the current is applied at multiple or only a single frequency. In one example, this includes first impedance values representing the impedance of the unaffected limb and second impedance values representing the impedance of the affected limb. However, alternatively, this may be impedance values representing the impedance of the entire body.

Once the one or more impedance values are determined, these are used by the processing system 102, together with a reference, to derive an indicator. This may be achieved in any one of a number of ways depending on the preferred implementation.

In one example, the indicator provides information relating to a subject parameter, such as an indication of fluid levels within the subject. This can be based on measured impedance values, or impedance parameters derived therefrom, such as the impedance at zero, characteristic and infinite frequencies. The indicator is typically in the form of a numerical value that is scaled relative to the reference, to thereby provide an indication of the relative value of the subject parameter, which can in turn be indicative of the presence, absence or degree of a condition, such as oedema, hydration levels, or the like.

This is typically achieved so that the indicator can be represented on a linear indicator, with the position of a pointer relative to an associated scale being indicative of the relative value of the subject parameter. In one example, the linear indicator can include thresholds at predetermined values representing ranges indicative of the presence or absence of a condition.

Thus, when the indicator is an oedema indicator, the numerical value and hence position of the pointer can be an indication of relative extracellular fluid levels in a limb, which can in turn be used to indicate the likely presence, absence or degree of oedema. In this regard, the skilled person will understand that any such indicator is not absolute, and that in practice this indicator is a mechanism for presenting information to a medical or healthcare professional, allowing them to interpret the measured data, and perform any diagnosis.

The oedema indicator can be based solely on the impedance measured for the "affected" limb. However, more typically the oedema indicator is based on an impedance ratio representing the impedance of the unaffected limb against the impedance of the affected limb. It will be appreciated that this is particularly advantageous as this provides a dimensionless numerical value that is easily scaled, and also takes into account inherent variations in absolute fluid levels within the subject.

It will be appreciated that in a healthy person, the impedance of both limbs will be similar and the impedance ratio will approximate 1.00. However, a population study of healthy subjects has found that slight differences in impedance have been recorded due to limb dominance in limbs. Hence the mean "normal" ratio used for comparison with a patient's ratio may be different depending on whether a patient's affected limb is the dominant one or not.

In any event, when the extracellular fluid in a limb increases, the impedance of that limb decreases. Consequently, the impedance ratio increases as the volume of extracellular fluid in the affected limb increases. Therefore as the impedance ratio increases the patient's condition is worsening.

In one example, the assessment of lymphoedema in a patient uses a comparison of the impedance ratio to a normal range of impedance ratios established in a healthy population. Patients whose impedance ratio is greater than three standard deviations away from the mean ratio are defined as having lymphoedema.

For ease of use and understanding by lymphoedema therapist and clinicians the impedance ratio is scaled, such that a pre-selected threshold value can be used to indicate a fluid level that is suggestive of the presence or absence of oedema. To achieve this, a reference population is used to scale the impedance ratio values such that three standard deviations from the mean (or another suitable value) is represented by a predetermined threshold value.

In one example the threshold value is set at a value of "10" and accordingly if the scaled index ratio has a value of greater than "10" then this is indicative of the presence of oedema in the affected limb. In the event that the scaled impedance ratio is between "-10" and "10" this indicates that this is within normal population ranges and therefore indicates that oedema is not typically present.

In the event that the scaled impedance ratio value is less than "-10", then this generally indicates that a problem with the measurement has occurred which requires further investigation. This may include for example, that the electrodes have been connected to the subject incorrectly, or that the affected limb has been incorrectly identified.

It will be appreciated however, that any scaling may be used, so that the value of "10" as being an oedema threshold is for the purpose of illustration only, and that in practice the value could be selected to be any suitable number. However, as will be appreciated by persons skilled in the art, the selection of a memorable integer value for the threshold makes the process of identifying the presence of oedema far easier.

If the indicator is used for other conditions, such as hydration levels, the position of the pointer can be used to indicate another subject parameter, such as overall fluid levels within the whole of a subject. In one example, the value is scaled relative to a reference obtained from relevant sample populations so that four standard deviations from a population mean has a value of "100". This allows the a user to rapidly evaluate any determined indicator value and more easily make a rapid assessment as to the hydration level of the subject relative to a standard normal population, which is in turn suggestive of dehydration or over hydration.

Once the indicator is determined, a representation of the indicator can be displayed to an operator at step 240.

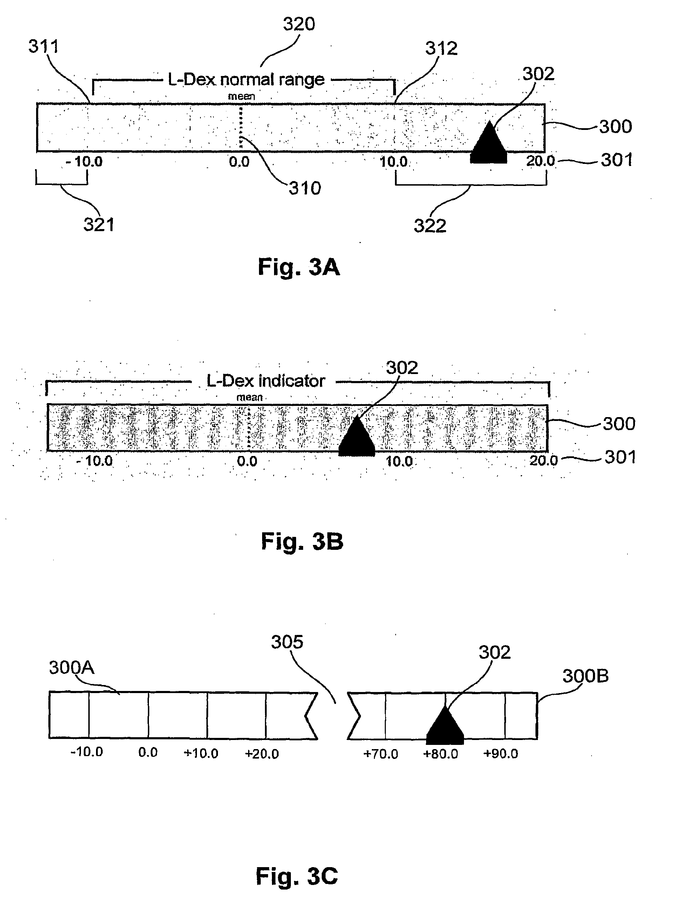

Example of such representations for oedema indicators will now be described with reference to Figures 3A and 3B.

In these examples, the representation is in the form of a linear indicator 300, having an associated scale 301 and a pointer 302. The position of the pointer 302 relative to the scale 301 is indicative of the subject parameter, which in this example is based on an impedance ratio representing a ratio of fluid levels determined for healthy and affected limbs of the subject.

In the example of Figure 3A, a reference population is available, and accordingly this is used to determine mean impedance and standard deviations for equivalent impedance ratio measurements made for a relevant sample population. The indicator representation also includes a mean indicator 310 representing the mean impedance ratio for the normal population, which is set to a value of "0" on the scale 301. The upper and lower thresholds are set to be three standard deviations from the mean 310, and are set to be positioned at "-10" and "+10" on the scale 301 respectively.

In use the lower and upper thresholds 311, 312 define a normal range 320, an investigation range 321, and an oedema range 322. The ranges can be indicated through the use of background colours on the linear indicator, so that for example, the normal range 320 is shaded green, whilst the investigation range 321 is unshaded, and the oedema range 322 is shaded red. This allows an operator to rapidly evaluate the positioning of the pointer 302 within the ranges, allowing for fast and accurate diagnosis of oedema based on the indicated fluid level information.

Thus, in the example of Figure 3A, the pointer 302 is positioned at the value of 16.6, placing the pointer 302 in the oedema range 322, indicating to the user that the fluid levels in the subject S are probably indicative of oedema in the affected limb.

In this example, the linear indicator extends up to a value of "20" as this is able to accommodate the determined value of 16.6. However, it will be appreciated that the linear indicator can be extended to any value required to accommodate the determined indicator value. To ensure that the linear scale remains clear, particularly if an extreme indicator value is to be displayed, the linear indicator 300 may include discontinuities, allowing the scale to be extended to higher values. An example of this is shown in Figure 3C, in which a discontinuity 305 is used to separate the linear indicator 300 into two portions 300A, 300B. In this example, the linear indicator portion 300A extends from "-10" to "+20", whilst the second linear indicator portion 300B extends from "+70" to "+90", thereby allowing an indicator value of "80" is to be displayed by appropriate positioning of the pointer 302 in the indicator portion 305B.

Whilst a linear indicator 300 is preferred as this easily demonstrates to the operator the potential degree of severity of any oedema, this is not essential, and alternatively the scale may be modified, particularly if an outlier indicator value is determined. Thus, for example, the linear indicator could include logarithmic scaling, or the like, over all or part of its length, to allow the determined indicator value to be displayed.

In the event that the indicator value is between "-10" and "+10", this indicates that the subject S is within the normal range 320 and that therefore they do not have oedema. Finally, in the event that the indicator value is below "-10", then the subject S is within the investigation range 321, indicating that the measurements need to be investigated further. In particular, it is extremely unlikely that the affected limb could have an impedance value significantly smaller than that of the unaffected limb, and accordingly, this indicates that in all likelihood there has been an error in the measurement, such as incorrect designation of the affect limb, or incorrect connection of electrodes.

In the example of Figure 3B, no reference population is available, and accordingly, the representation does not includes a mean 310 or lower or upper thresholds 311, 312. In this instance, the indicator value is still scaled, but rather than basing this on a mean and standard deviation of a reference population, this is achieved using default standard values for the mean and the standard deviation.

As a result an oedema indicator value of above "10" is still indicative of oedema, but may be a less reliable indicator than if the reference is available. To take this into account, the thresholds 311, 312, and hence the specific ranges 320, 321, 322, are excluded from the representation, highlighting to the operator that the scaled subject parameter value is indicative but not definitive of the subject's oedema status.

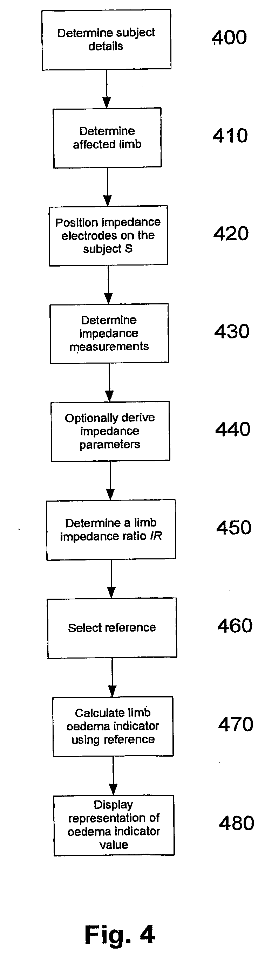

An example of the process for determining an indicator for unilateral limb oedema will now be described in more detail with reference to Figure 4.

In this example, at step 400 subject details are determined and provided to the processing system 102. The subject details will typically include information such as limb dominance, details of any medical interventions, as well as information regarding the subject as the subject's age, weight, height, sex, ethnicity or the like. The subject details can be used in selecting a suitable reference normal population, as well as for generating reports, as will be described in more detail below.

It will be appreciated that the subject details may be supplied to the processing system 102 via appropriate input means, such as the I/O device 105. Thus, each time a subject measurement is performed this information can be input into the measuring device 100.

However, more typically the information is input a single time and stored in an appropriate database, or the like, which may be connected as a peripheral device 104 via the external interface 103. The database can include subject data representing the subject details, together with information regarding previous oedema indicators, baseline measurements or impedance measurements recorded for the subject.

In this instance, when the operator is required to provide subject details, the operator can use the processing system 102 to select a search database option allowing the subject details to be retrieved. This is typically performed on the basis of a subject identifier, such as a unique number assigned to the individual upon admission to a medical institution, or may alternatively be performed on the basis of name or the like. Such a database is generally in the form of an HL7 compliant remote database, although any suitable database may be used.

In one example, the subject can be provided with a wristband or other device, which includes coded data indicative of the subject identifier. In this case, the measuring device 100 can be coupled to a peripheral device 104, such as a barcode or RFID (Radio Frequency Identification) reader allowing the subject identifier to be detected and provided to the processing system 102, which in turn allows the subject details to be retrieved from the database. The processing system 102 can then display an indication of the subject details retrieved from the database, allowing the operator to review these and confirm their accuracy before proceeding further.

At step 410 the affected limb, or "at risk" limb, is determined. This may be achieved in any one of a number of ways depending on the preferred implementation. Thus, for example, the affected limb can be indicated through the use of appropriate input means, such as the I/O device 105. Alternatively this information can be derived directly from the subject details, which may include an indication of the affected limb, or details of any medical interventions performed, which are in turn indicative of the affected limb.

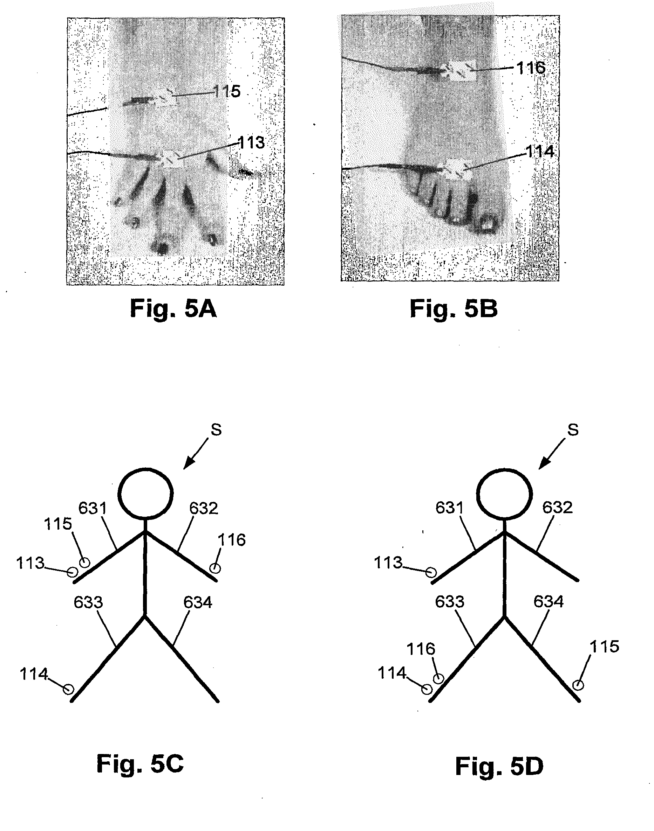

At step 420 an operator positions the electrodes on the subject S, and connects the leads 123, 124, 125, 126, to allow the impedance measurements to be performed. The general arrangement is to provide electrodes on the hand at the base of the knuckles and between the bony protuberances of the wrist, as shown in Figure 5A, and on the feet at the base of the toes and at the front of the ankle, as shown in Figure 5B. The configurations shown in Figures 5C and 5D allow the right arm 631 and the right leg 633 to be measured respectively, and it will be appreciated that equivalent arrangements can be used to measure the impedance of the left leg and left arm.

It will be appreciated that this configuration uses the theory of equal potentials, allowing the electrode positions to provide reproducible results for impedance measurements. For example when current is injected between electrodes 113 and 114 in Figure 5C, the electrode 116 could be placed anywhere along the left arm 632, since the whole arm is at an equal potential.

This is advantageous as it greatly reduces the variations in measurements caused by poor placement of the electrodes by the operator. It also greatly reduces the number of electrodes required to perform segmental body measurements, as well as allowing the limited connections shown to be used to measure each of limbs separately.

However, it will be appreciated that any suitable electrode and lead arrangement may be used.

At step 430 the impedance of the affected and contralateral limbs are measured. This is achieved by applying one or more current signals to the subject and then measuring the corresponding voltages induced across the subject S. It will be appreciated that in practice the signal generator 111, and the sensor 112, return signals to the processing system 102 indicative of the applied current and the measured voltage, allowing impedances to be determined.

Following this a limb impedance ratio *IR* is determined, and the manner in which this is achieved will depend on the nature of the impedance measurements performed.

In the case of BIS analysis, the impedance ratio can be based on impedance parameter values, such as values of the impedance at zero, characteristic or infinite frequencies ($R_0$, $Z_c$, $R_\infty$).

Accordingly, at step 440, these values can be derived based on the impedance response of the subject, which at a first level can be modelled using equation (1), commonly referred to as the Cole model:

$$Z = R_\infty + \frac{R_0 - R_\infty}{1 + (j\omega\tau)} \tag{1}$$

where:

$R_\infty$ = impedance at infinite applied frequency,
$R_0$ = impedance at zero applied frequency,
$\omega$ = angular frequency,
$\tau$ is the time constant of a capacitive circuit modelling the subject response.

However, the above represents an idealised situation which does not take into account the fact that the cell membrane is an imperfect capacitor. Taking this into account leads to a modified model in which:

$$Z = R_\infty + \frac{R_0 - R_\infty}{1 + (j\omega\tau)^{(1-\alpha)}} \tag{2}$$

where: $\alpha$ has a value between 0 and 1 and can be thought of as an indicator of the deviation of a real system from the ideal model.

The value of the impedance parameters $R_0$ and $R_\infty$ may be determined in any one of a number of manners such as by:

- solving simultaneous equations based on the impedance values determined at different frequencies;
- using iterative mathematical techniques;
- extrapolation from a "Wessel plot";
- performing a function fitting technique, such as the use of a polynomial function.

In any event, in this example, the impedance ratio can be determined at step 450 using the equation:

$$IR = \frac{R_0 ul}{R_0 al} \tag{3}$$

where:

> $IR$ is the impedance ratio
> $R_0ul$ is the impedance of the unaffected limb at zero frequency
> $R_0al$ is the impedance of the affected limb at zero frequency

In this example, the impedance parameter value $R_0$ is used as this is generally indicative of extra-cellular fluid levels in the respective limb, which are in turn indicative of the presence, absence or degree of oedema. However, other impedance parameters can be used.

Alternatively, in the case of a BIA analysis, the impedance ratio is generally given by the actual impedances measured at a single low frequency, such as 50 kHz or below, as this is a good approximation of $R_0$, and hence is generally indicative of extra-cellular fluid levels. However, alternatively, the impedance ratio can be based on reactance, resistance or phase measurements, or a combination thereof:

$$IR = \frac{Zul}{Zal} \tag{4}$$

where:

> $Zul$ is the measured impedance of the unaffected limb
> $Zal$ is the measured impedance of the affected limb

At step 460 a reference is selected. The reference is typically derived from equivalent measurements made on a normal population (subject's not suffering from oedema) that is relevant to the subject under study. Thus, the normal population is typically selected taking into account factors such as medical interventions performed, ethnicity, sex, height, weight, limb dominance, the affected limb, or the like.

Therefore if the test subject has unilateral lymphoedema of the dominant arm and is female then the normalised data drawn from the normal population database will be calculated from the dominant arm impedance ratio measurements from female subjects that are present in the normal population database.

Accordingly, at this stage the processing system 102 typically accesses reference populations stored in the database, or the like. This may be performed automatically by the processing system 102 using the subject details. Thus for example, the database may include a look-up table that specifies the normal population that should be used given a particular set of subject details. Alternatively selection may be achieved in accordance with predetermined rules that can be derived using heuristic algorithms based on selections made by medically qualified operators during previous procedures. Alternatively, this may be achieved under control of the operator, depending on the preferred implementation.

It will be appreciated by persons skilled in the art that operators may have their own reference stored locally. However, in the event that suitable references are not available, the processing system 102 can be used to retrieve a reference from a central repository, for example via an appropriate server arrangement. In one example, this may be performed on a pay per use basis.

Alternatively, in the event that a suitable reference is not available predetermined standard reference values may be used.

In one example, the reference values are based on the mean impedance ratio, and an impedance ratio value three standard deviations from the mean impedance ratio for the normal population, and example values are set out below. However it will be appreciated that different values can be used as appropriate and that these values are for illustration only:

> $\mu$ = 1.00
> $3\sigma$ = 1.102

Whilst the use of predetermined reference values allows an oedema indicator to be derived it will be appreciated that this is not necessarily indicative of the presence, absence or degree of oedema and therefore the indicator may be displayed independent of any thresholds, as will be described in more detail below.

At step 470 a limb oedema indicator is generated utilising the reference. As described above, this is typically achieved by scaling the impedance ratio using the reference population, and in particular using the mean and the standard deviation of the reference.

As described above, this is performed so that the value of three standard deviations corresponds to a memorable value. To achieve this, in one example, the transformation of the impedance ratio to an oedema indicator value is governed by the following formula:

$$L-Dex = \frac{sf \times (IR - \mu)}{3\sigma - \mu} \tag{5}$$

where:

> *L-Dex* is the oedema indicator
> *IR* is the impedance ratio
> $\mu$ is the mean impedance ratio for a reference population
> $3\sigma$ is impedance ratio value that is three standard deviations from the mean population impedance ratio value
> *sf* is the scaling factor

The scaling factor is selected so that the thresholds correspond to a memorable value, and in particular, the scaling factor is typically an integer value, and more typically a multiple of ten. Thus, in one example, the scaling factor is set to a value of "10", so that the threshold occurs at "10". As a result, an oedema indicator value of greater than "10" is indicative of oedema, whilst a value of below "10" is used to indicate an absence of oedema.

In this example, for a subject S whose at-risk arm is the dominant arm and has an impedance ratio of 1.207, the subject's impedance ratio is scaled using a suitable normal population. For the purpose of this example, the normal population has a mean impedance ratio value of 1.037 and a three standard deviation value of 1.139. This leads to an oedema indicator of:

$$L\text{-}Dex = (1.207\text{-}1.037) \times 10 / (1.139\text{-}1.037) = 16.6$$

In any event once the oedema indicator value has been calculated at step 470 a representation of the oedema indicator value can displayed to the user at step 480, with the oedema indicator value being optionally recorded in a database, for example as part of the subject details. The oedema indicator can be stored together with any relevant information, such as the time and date on which the measurement was performed, details of the operator of the measuring device 100, or the like. This allows for the measured oedema indicator to be subsequently retrieved and used in tracking the development and/or progression of the oedema, allowing the effectiveness or need for treatment to be evaluated.

Display of the representation may be achieved in a number of ways, such as by presenting the representation on a suitable display, for example, using the I/O device 105, or alternatively by providing the representation in a hard copy form using an appropriate printer, although any suitable technique may be used.

Whilst any suitable form of representation may be used, in one example, the representation is presented in such a way to provide guidance to any one or more of the following questions:

> 1. Does the patient have lymphoedema?
> 2. How bad is the lymphoedema relative to a normal person?
> 3. Has the patient's condition got better or worse since the last measurement?
> 4. What is the overall change in the patient's condition from their starting point?

In one example, this is achieved by displaying the oedema indicator as a linear scale, similar to those described above with respect to Figures 3A and 3B.

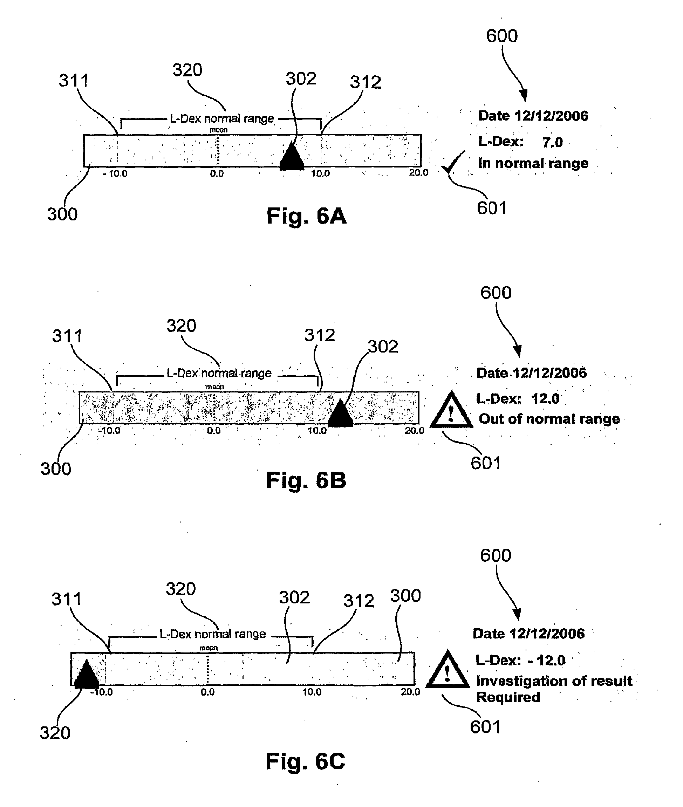

Further examples of the linear scales are shown in Figures 6A to 6C, which respectively show a measurement within the normal range 320, a measurement within the oedema range 322 and a measurement within the investigation range 321.

In these examples, the representations include additional information in the form of textual information 600 and an icon 601. In this example, the textual information is indicative of the date of the measurement, the oedema indicator (*L-Dex*) value and whether or not the oedema indicator value is in the normal range 320. Additionally the icon 601 is indicative of whether or not the oedema indicator value is in the normal range. In this example, the icon 601 is in the form of a tick in the event that the oedema indicator value is within the normal range, and in the form of an alert icon, shown in Figures 6B and 6C if the oedema indicator is out of normal range 320, in either the investigation or oedema ranges 321, 322.

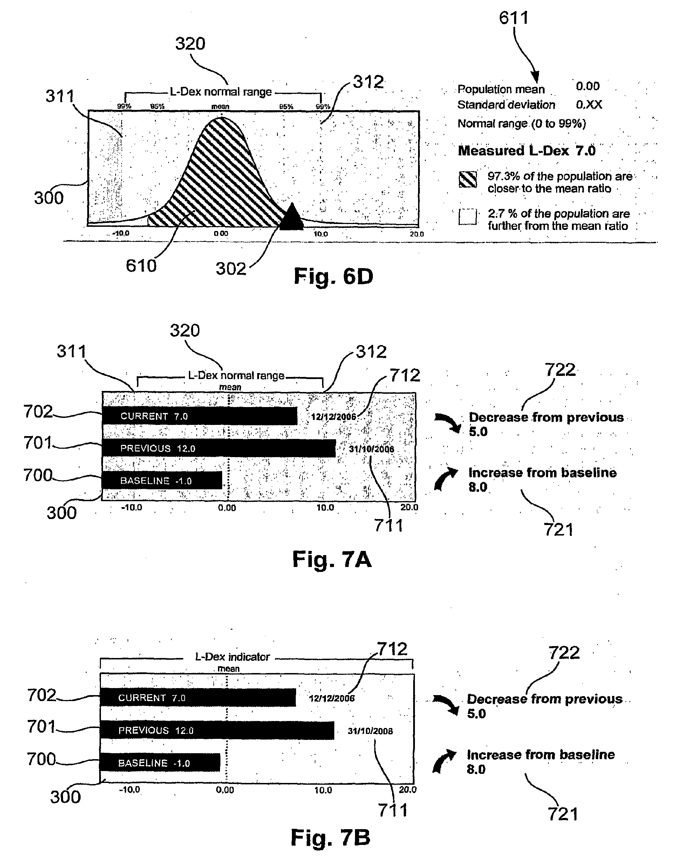

A further example is shown in Figure 6D in which the distribution of the normal population is displayed on the linear indicator 300 as a Gaussian distribution 610. In this example, additional textual information 611 includes information such as the values of the population mean and standard deviation, and the relative positioning of the subject's measured oedema indicator value relative to the population distribution.

Accordingly, it will be appreciated that the above described representations allow operators to easily access one or more of the following:

- values of the normal (mean) oedema indicator and oedema indicator normal range;
- the date of the measurement;
- the subject's oedema indicator value;
- an indication of when a subject's impedance ratio is in the normal range 320, the investigation range 321, or the oedema range 322; and,
- an indication of whether clinical assessment of the subject is required for example when a subject's oedema indicator is in the investigation range.

It will be appreciated that the representations may also include additional features. Thus, for example, the linear indicator 300 can contain "overflow" buffers at either end of the scale that contain a compressed scale that can deal with oedema indicator numbers of high values. Additionally, or alternatively, the linear indicator can be automatically scaled to fit the relevant information on the display. Displayed reports can also contain patient identification, date of measurement, titles, or the like.

In further examples, the representations can be modified to indicate how the subject's oedema indicator is varying relative to a baseline, as shown for example in Figures 7A and 7B.

In order to achieve this, the processing system 102 accesses a baseline and optionally other previous oedema indicator values measured for the subject S from the database.

Typically, the baseline oedema indicator is created from an oedema indicator measurement that has significance in the treatment history of the patient. A common baseline in use might be an oedema indicator measurement made on a patient suffering from lymphoedema before they start a course of management therapy. This measurement allows the practitioner to gauge accurately how much the patient has improved from the start of their treatment to the present measurement. Baseline measurements may also be made pre-surgery and prelymphoedema in which case the baseline oedema indicator establishes the "normal" healthy oedema indicator for the individual patient and can be used thereafter as a benchmark from which to monitor progress of the patient. Baselines can also be set using a single measurement or be created from the average of a number of measurements specified by the user.

In any event, in this example, the pointer 302 on the linear indicator 300 is replaced by indicator bars 700, 701, 702 representing baseline, previous and current values for the oedema indicator respectively. Each one of the indicator bars 700, 701, 702 may have an associated date displayed as shown at 711, 712, indicating the date at which the corresponding measurement was made (only two shown in this example for clarity).

In addition to this, the representation can include change indicators 721, 722, indicating the change in the oedema indicator between the baseline and previous measurements, and between the previous and current measurements, as shown. In this example, the change indicators 721, 722 represent the difference between the present and previous oedema indicator values as factional numbers to 1 decimal point with an indication of increase or decrease in value. However, any suitable representation may be used.

In the event that a normal population is used in the scaling of the oedema indicator, then the linear indicator 300 again includes thresholds 311, 312, defining the normal range 320, as shown in Figure 7A. However, if a normal population is not available, then as in previous examples, predetermined reference values are used, and the thresholds 311, 312, and hence the normal range 320, are not displayed, as shown in Figure 7B.

It will be appreciated that this allows the oedema indicator to be displayed in a manner that conveys how this ratio has changed from an established baseline ratio for that patient.

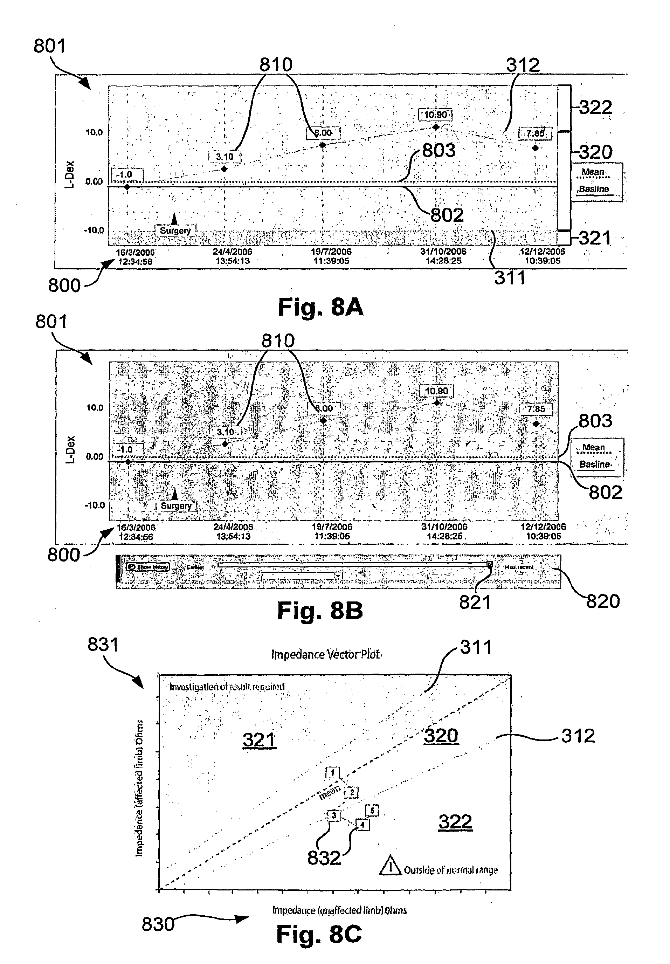

Further alternative representations of the change in oedema indicator values over time are shown in Figures 8A and 8C.

In Figures 8A and 8B, the oedema indicator is represented as a progression chart documenting the longitudinal progress of the oedema indicator over time. In this example, the progression chart includes an x-axis 800 displaying the time and date on which measurements were made, and a y-axis 801 including oedema indicator values.

The value of the oedema indicator at a baseline reading can also be indicated at 802, together with a mean for the reference normal population 803. The actual measured oedema indicator values are then displayed on the graph as shown generally at 810.

In the event that a reference normal population is available, then the threshold values representing three standard deviations from the mean can be displayed as shown at 311, 312, with the normal range, intervention range and oedema range being indicated through the use of coloured background regions on the chart, shown generally at 320, 321, 322. In the event that no reference normal population is available, the thresholds 311, 312, and regions 320, 321, 322 are omitted as shown in Figure 8B.

In general the processing system 102 can allow a selection mechanism to be used to select a range of measurements from the subject's details stored in the database, for display. This selection mechanism typically uses default values for displaying all measurements in the subject's database, such as the five most recent measurements and the ten most recent measurements.

In one example, the processing system 102 can provide the user with an input means, such as a slider shown at 820 in Figure 8B, that allows the common history mechanism to be populated or de-populated as a user "slides" a pointer 821 from an earliest to more recent measurements. This allows an operator to scan quickly (or measurement by measurement) through the dated and time stamped measurements within a subject's database, in turn allowing quick review of the progression of the oedema. This also allows an operator to select only the last five measurements out of a total of 20 to be displayed which can help bypass the "cramped" look of a history chart with too many data points if the whole history of the patient were to be displayed.

In addition to displaying the oedema indicator values, any other measured numerical parameters can also be presented on a similar chart showing the change in measured values over time. This can include impedance measurements, as well as values of impedance parameters, such as the impendance at zero, characteristic or infinite frequencies.

A further alternative is shown in Figure 8C. In this example, the oedema indicator is displayed as part of an impedance vector plot.

In this example, the chart includes an x-axis 830 representing the impedance of the unaffected arm and a y-axis 831 representing the impedance of the affected arm, with the oedema indicator values being represented by the points 832.

Again, in the event that a normal population is available as a reference, the thresholds 311, 312 can be displayed to define the normal range 320, the investigation range 321 and the oedema range 322.

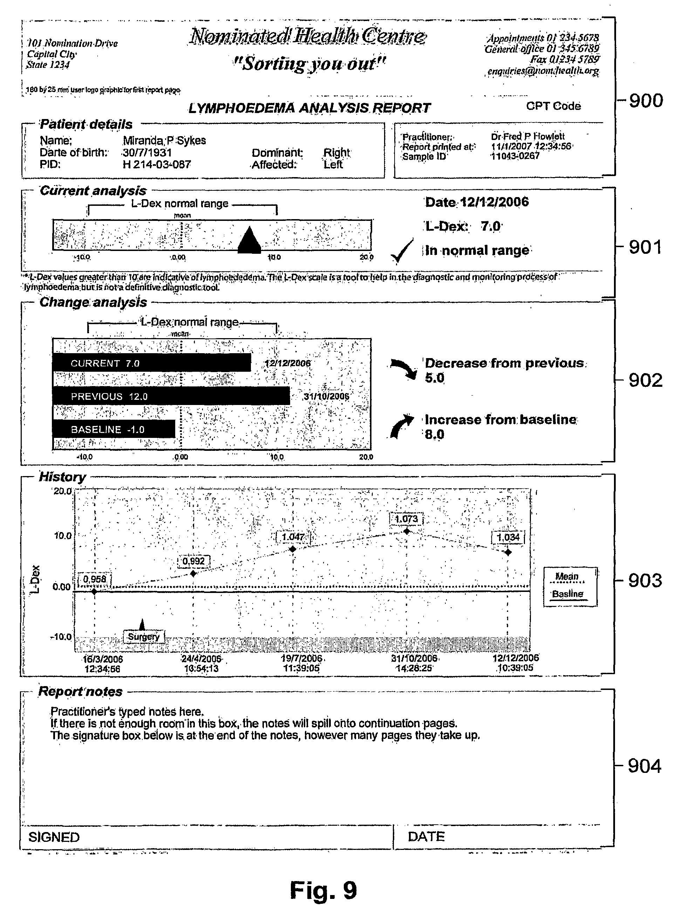

As shown in Figure 9 a further variation is to provide the representation of the oedema indicator as part of a report containing one or more of the above described representations.

In general the report would be configured to fit on one A4 or USA letter page, thereby allowing the report to act as a standard unilateral oedema report.

In this example, the printable report incorporates a number of sections shown generally at 900, 901, 902, 903, 904. In use, the report is generated by the processing system 102 using a standard template that is populated using data regarding the subject which has been retrieved from the database.

In this example, the report includes a header section 900 outlining details of the operator performing the oedema detection measurements, the subject, the entity employing the operator, and any CPT coding and other information required by the USA reimbursement policy.

A current analysis section is provided at 901 to display the current oedema indicator using a representation similar to that described above with respect to Figures 6A to 6C.

A change analysis section is provided at 902 to display changes in the oedema indicator using a representation similar to that described above with respect to Figures 7A and 7B.

A history analysis section is provided at 903 to the history of the oedema indicator using a representation similar to that described above with respect to Figures 8A and 8B.

Finally a footer section 904 is provided to allow any additional notes and an operator signature to be provided.

Accordingly, it will be appreciated that the report can be used to provide the subject with an analysis of both the current status and the progression of oedema, as well as to allow the reimbursement of expenditure on both measurement and treatment of the condition.

It will be appreciated that the techniques described above may be used to allow a range of different indicators relating to different subject parameters, and hence allowing diagnosis of different conditions, to be determined and displayed.

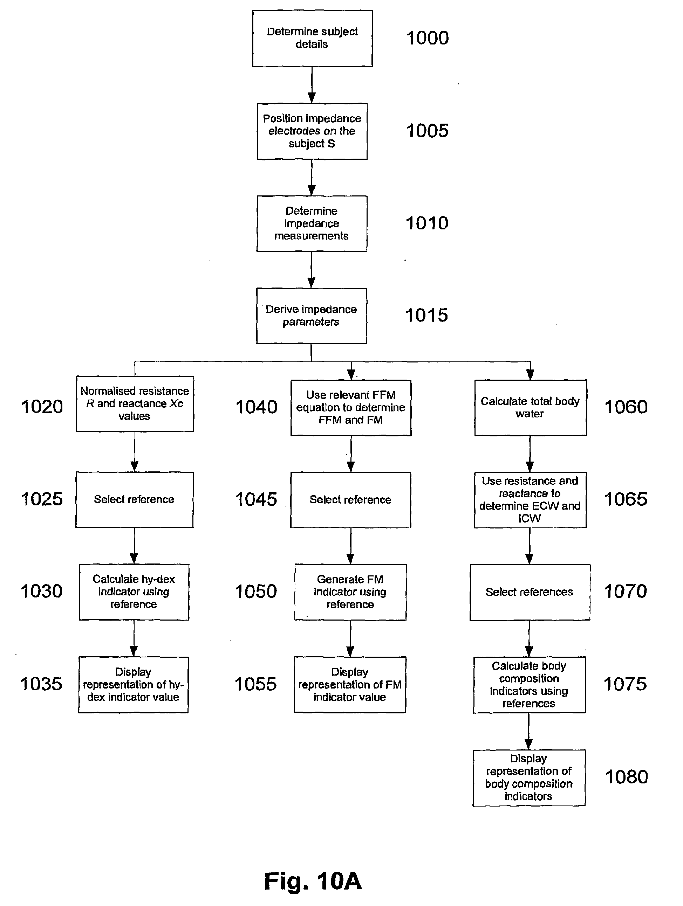

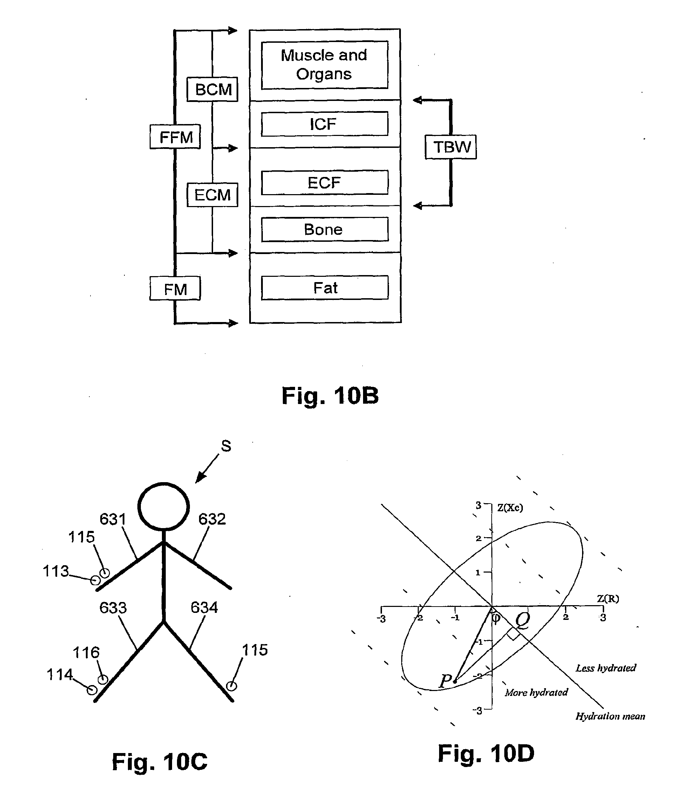

Examples of alternative indicators will now be described with reference to Figures 10 to 14.

In this regard, Figure 10A is a flow chart of a process for determining a number of other indicators including a hydration (hy-dex) indicator, a fat mass (FM) indicator, and other body composition indicators.

In this regard, the hy-dex indicator is an indication of the hydration levels of a subject, scaled relative to the normal hydration levels of a sample reference population, thereby allowing memorable values to be indicative of whether the subject is dehydrated, or over hydrated.

The FM indicator is used to indicate the fat versus fat-free mass for the subject, whilst the body composition indicators are used to provide indications of various body composition parameters. This can include for example, providing indications of intra- and extra-cellular fluid levels, and total body water.

The relationship between the body composition indicators is shown in Figure 10B, which is an illustration of a composition model for a body. In this example, the model has a number of compartments including the bone, the fat, the muscle, organs, fluid surrounding these tissues and fluid contained inside these tissues.

As shown, the fat-free mass (FFM) is indicative of the mass of all components other than the FM component.

The Body Cell Mass (BCM) (sometimes referred to as Active Tissue Mass (ATM)) is the total mass of all the cellular elements in the body which constitute all the metabolically active tissue of the body, and this includes muscle and organ tissue, and intracellular fluid. The Extracellular Mass (ECM) contains all the metabolically inactive parts of the body, such as bone and blood plasma, and therefore includes the extracellular fluid (ECF). The ECM parameter has been used to describe changes in weight that may not be attributed to a gain in fat mass or fat-free mass.

In the normally nourished individual, muscle tissue accounts for approximately 60% of the BCM, organ tissue accounts for 20% of BCM, with the remaining 20% made up of red cells and tissue cells. The BCM also contains the large majority (98-99%) of the body's potassium. The BCM provides a way to document the nutritional status of an individual and track the calorific requirements of a person by using an equation that calculates the basal metabolic rate of individual using the BCM.

Finally the BMI is an index defining weight ranges and was developed by the National Institutes of Health (NIH). The body mass index (BMI) relates body weight to height and is obtained by dividing a person's weight in kilograms (kg) by their height in meters (m) squared. The NIH now defines normal weight, overweight, and obesity according to the BMI rather than the traditional height/weight charts. Since the BMI describes the body weight relative to height, it correlates strongly (in adults) with the total body fat content. Overweight is a BMI of 25 or more for women men, according to the NIH. Obesity is a BMI of 30 and above, according to the NIH. A BMI of 30 is about 30 pounds overweight. Some very muscular people may have a high BMI without undue health risks.

In this example, at step 1000 various subject details are determined. The subject details determined may vary depending on the indicator to be determined and the preferred implementation but would generally include information such as the subject's total body weight, sex, age, height, and an ethnicity, and may be achieved using techniques similar to that described above with respect to Figure 4.

At step 1005 the impedance electrodes are positioned on the subject S. In this instance the measurements are typically performed as whole body measurements and this therefore involves positioning drive electrodes 113, 114 on the wrist and ankle of the subject's arm and leg 631, 633, with sense electrodes 114, 115 being positioned inwardly of the drive electrodes 113, 114 on the same wrist and ankle, as shown in Figure 10C.

At step 1010 impedance measurements are performed, and this may include performing single low frequency (typically 50 kHz or below) BIA measurement, or multiple frequency BIS measurements, substantially as described above.

At step 1015 impedance parameters are derived for the subject's entire body. The impedance parameters determined will vary depending on the indicator being generated. Thus, for example, this could include the measured impedance value, particularly in the case of BIA measurements. However, in the case if BIS, this may also include deriving parameters such as values of the impedance at zero, characteristic or infinite frequencies ($R_0$, $Z_c$, $R_\infty$), using the techniques described above.

In the event that a hy-dex indicator is to be determined, at step 1020 impedance values are measured as complex numbers, allowing values of resistance R and reactance $Xc$ to be normalised based on the subject's height $h$ by calculating $R/h$ and $Xc/h$.

As step 1025 a reference is determined based on measured impedance values for a reference population that is relevant for the subject. The reference may be determined in any manner, as described above for example, with respect to Figure 4.

In one example, the reference is based on mean and standard deviation values derived for normalized resistance and reactance measurements ($(R/h)_{mean}$, $(Xc/h)_{mean}$, $(R/h)_{std.dev}$ and $(Xc/h)_{std.dev}$, for a normal population group selected based on the patients sex, age and BMI.

For each of these groups a normalised resistance ($R/h$) vs reactance ($\underline{Xc/h}$) plot, known as an $RXc$ score graph 95% tolerance ellipse, can be determined based on the techniques described in Piccoli et al 2002 "Impedance vector distribution by sex, race, body mass index and age in the United States: standard reference intervals as bivariate Z scores." Nutrition 18: 153-167. This plot allows values $(R/h)_{mean}$, $(Xc/h)_{mean}$, $(R/h)_{std.dev}$ and $(Xc/h)_{std.dev}$, to be determined for the respective group, although it will be appreciated that these values may be derived using any suitable technique.

At step 1030, the hy-dex indicator value is determined by calculating the normalised vector distance from the population mean using the following equations:

$$R_v = [ \ (R/h) - (R/h)_{mean} \ ] \ / \ (R/h)_{std.dev} \qquad (6)$$

$$Xc_v = [ \ (Xc/h) - (Xc/h)_{mean} \ ] \ / \ (Xc/h)_{std.dev}$$

and then scaled relative to a scaling factor, using the equation:

$$hy - dex = \frac{sf \times |R_v, X_{cv}| \sin(\varphi)}{4\sigma} \qquad (7)$$

Where $\varphi$ is the angle between the patients normalised measurement vector and the mean hydration line, as shown in Figure 10D.

Represented graphically, if $R_v$ and $X_{cv}$ are plotted on a Picolli plot as a point $P$, as shown in Figure 10D, the point $P$ forms the $RX_c$ score graph. The line $R=-X_c$ corresponds to a normal mean hydration, with points above and to the right being less hydrated and points below and to the left are more hydrated. The major axis radius (length 3.14, from Piccoli et al) of the 95% tolerance ellipse represents a hydration state two standard deviations from the mean. Thus one standard deviation is half the major radius. Hydration standard deviations are shown dotted in the diagram.

Accordingly, to calculate the Hy-Dex indicator value, the perpendicular distance $PQ$ to the mean hydration line is calculated and given a positive sign if it is below and to the left or a negative sign if it is above and to the right. This is then scaled using equation (8):

$$Hy\text{-}Dex = sf * length(PQ) / (major\ diameter\ of\ 95\%\ tolerance\ ellipse) \qquad (8)$$

where: $sf$ is a scaling factor

It will be appreciated that the scaling factor can therefore be selected so that extremes correspond to a memorable value, and in particular, the scaling factor is typically an integer value, and more typically a multiple of ten. Thus, in one example, the scaling factor is set to a value of "100", so an indicator value of 100 represents 4 standard deviations from the mean hydration level for a relevant normal population.

Thus, the hy-dex indicator can be thought of as depicting a patient's movement away from the average mean hydration level, with each single standard deviation from the mean, corresponding to 25 hy-dex units, as shown in Table 1.

Table 1

| Standard deviations | Hy-dex score |
|---|---|
| plus 4 | + 100 |
| plus 3 | +75 |
| plus 2 | +50 |
| plus 1 | +25 |
| Mean 0 | 0 |
| minus 1 | -25 |
| minus 2 | -50 |
| minus 3 | -75 |
| minus 4 | -100 |

It will be appreciated that in the unlikely event that a subject has a hydration value that exceeds more than 4 standard deviations away from the mean, this can simply be indicated by an indicator value 100, which in any event represents an extreme hydration/dehydration state.

In any event, this allows an indicator indicative of the relative hydration level of a subject to be displayed. In this regard, the value displayed is a relative fluids level, derived from normalised resistance and reactance values.

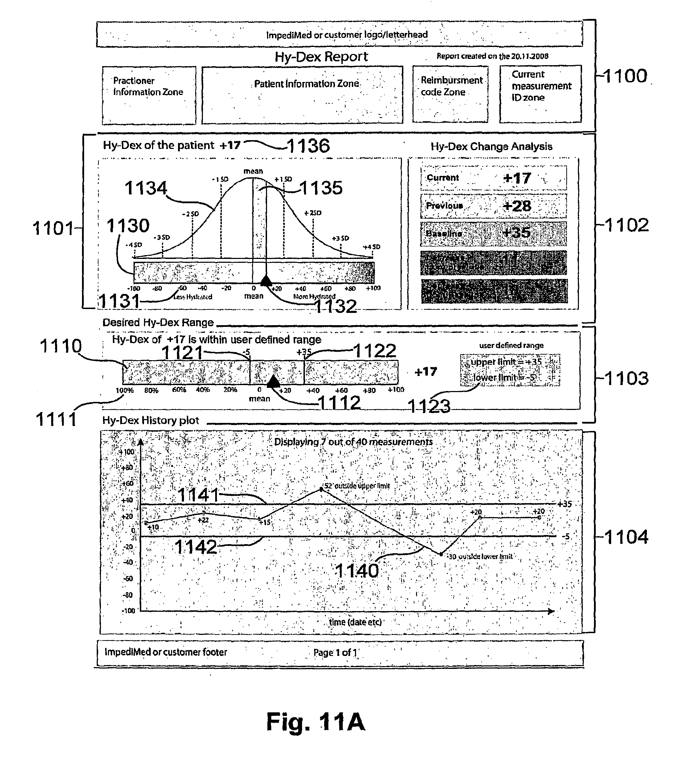

An example of a hy-dex report incorporating hy-dex indicators will now be described with reference to Figures 11A to 11C.

In this example, the report includes a header section 1100, outlining information such as the operator performing the measurement process, details of the subject, reimbursement code information, measurement ID zone information or the like. This is similar to the header section 900 of the Lymphodema report of Figure 9, and will not therefore be described in any further detail.

The report also includes a first and second hy-dex indicator sections 1101, 1103, and a hy-dex change analysis summary section 1102.

In this example, the first hy-dex indicator section 1101 includes a linear hy-dex indicator 1130 having an associated scale 1131 and a pointer 1132. In one example, a Gaussian distribution curve 1134 is provided for the sample population, which highlights the mean hydration value for the relevant sample population, and the values of the standard deviations therefrom. In this example, an area under the distribution curve is shaded 1135 to highlight the difference between the patients hydration indicator value indicated by the pointer 1132 and the sample population mean hydration. In this example, a visual indication of the numerical hy-dex indicator value is also displayed at 1136.

It will be appreciated from this, that presenting the indicator value in this fashion allows a medical practitioner to rapidly assess a subject's fluid levels and hydration status, thereby allowing the practitioner to determine if the subject is hydrated or dehydrated. Thus, for example, a hy-dex indicator value of "0" indicates that the subject has normal hydration levels, whilst a positive or negative value represents an overhydrated or dehydrated state. The magnitude of the indicator value also represents the degree of hydration, with a value of "+100+ or "-100" indicating an extreme state. This allows a medical practitioner to rapidly assess what if any treatment is required.

Similarly the second hy-dex indicator section 1103 includes a linear hy-dex indicator 1110, having an associated scale 1111 and a pointer 1112. In addition to this, a defined range including an upper and lower limit 1121, 1122 is indicated, with the upper and lower limit thresholds being indicated separately at 1123. In this example, the upper and lower limit are set by a medical practitioner, and may be used for rapidly assessing when treatment or intervention is required, or could be set as a guide to the subject as to desirable hydration levels, and need not be based on reference population values.

In this example, the report also includes a hy-dex progression chart section shown generally at 1104, which provides a progression chart similar to those described above with respect to Figure 8.

In this example, the progression chart includes graph 1140 indicating the subject's previous hydration values, together with indications of the upper and lower limits 1141, 1142. The previous values would typically be retrieved from previous measured values for the subject from a remote database, patient record, or the like, in a manner similar to that described above.

Whilst the hy-dex indicators described form part of a hy-dex report, this is not essential, and it will be appreciated that the hy-dex indicators could be presented to a user in any suitable manner.

Examples of further variations of the hy-dex indicators shown in 11B and 11C.

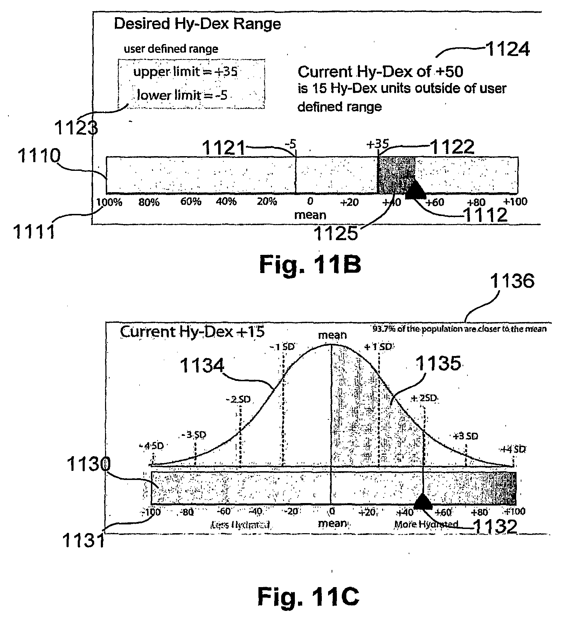

In this regard Figure 11B shows the second indicator in a situation in which the subject's hydration is outside the user defined range indicated by the thresholds 1121, 1122. In this instance an indication 1124 is provided indicating the degree to which the subject's hy-dex value is out of the user defined range, and this can also be indicated by shading of the hy-dex indicator 1110 at 1125.

In Figure 11C an example of the first indicator 1101 is shown in which an additional indication 1136 is provided indicating the percentage of the population that are closer to the mean hydration levels of the sample population than the subject S.

It will be appreciated that the hy-dex indicators described above are similar to the oedema indicators described with respect to Figures 3A to 3C and Figures 6A to 6D, and Figures 8A and 8B. Furthermore, variations similar to those described above with respect to oedema indicators could also be implemented, and these will not be described in any further detail for clarity purposes.

Accordingly, whilst the hy-dex indicators described form part of a hy-dex report, this is not essential, and it will be appreciated that the hy-dex indicators could be presented to a user in any suitable manner.

In addition to allowing a hy-dex indicator to be determined, the process of Figure 10A can additionally/alternatively allow FM indicators to be determined and displayed.

In this example, at step 1040 the impedance parameters derived at step 1015 can be used together with a fat free mass equation to determine the users fat free mass and hence fat mass.

This can be achieved utilising the equations set out below. The equation used depends on the subject gender, age, and obesity levels, as follows:

General Female:

$$\text{FFM} = \frac{0.734 * \text{ht}^2}{R} + (0.116 * \text{wt}) + (0.096 * \text{Xc}) - 4.03$$

General Male:

$$\text{FFM} = \left( \frac{0.734 * \text{ht}^2}{R} + (0.116 * \text{wt}) + (0.096 * \text{Xc}) - 4.03 \right) - 0.878$$

18

Obese Female:

$$FFM = (0.00091186 * ht^2) - (0.01466 * R) + (0.2999 * wt) - (0.07012 * age) + 9.37938$$

Obese Male:

$$FFM = (0.000885 * ht^2) - (0.02999 * R) + (0.42688 * wt) - (0.07002 * age) + 14.52435$$

Child:

$$FFM = \frac{0.81 * ht^2}{R} + 6.86$$

where:

ht = subject height
wt = subject weight

The Fat Mass is then given by:

$$FM = wt - FFM$$

Alternatively, FM can be calculated from BIS measurements. In this case, FM is calculated from the extracellular and intracellular water (ECW and ICW) and the following equations may be used.

$$ECW = K \left( \frac{\sqrt{wt}\ ht^2}{R_e} \right)^{\frac{2}{3}}$$

Note: K includes body density constant, body proportion coefficient and resistivity coefficient for ECW.
The following equation is then solved where $V_{ICW}$ and $V_{ECW}$ are the volumes of ECW and ICW respectively.

$$\left( 1 + \frac{V_{ICW}}{V_{ECW}} \right)^{\frac{5}{2}} = \left( \frac{R_e + R_i}{R_i} \right) \left( 1 + \frac{k_\rho V_{ICW}}{V_{ECW}} \right)$$

Where

$$R_i = \frac{R_e R_\infty}{R_e - R_\infty} \qquad k_\rho = \frac{\rho_{ICW}}{\rho_{ECW}}$$

This result is a ratio of the volumes of ICW/ECW. ICW can then be calculated.

$$ICW = \frac{V_{ICW}}{V_{ECW}} * ECW$$

From this the total body water (TBW) is calculated and from this the FFM and FM respectively.

$$TBW = ECW + ICW$$

$$FFM = TBW / 0.732$$

$$FM = wt - FFM$$

At step 1045 a reference is again selected and this is typically achieved using nominal reference population data, such as the data in the table 2 below.

Table 2

| Age(years) | Male Healthy % FM | Female Healthy % FM |
|---|---|---|
| 7 | 13-20% | 15-25% |
| 8 | 13-21% | 15-26% |
| 9 | 13-22% | 16-27% |
| 10 | 13-23% | 16-28% |
| 11 | 13-23% | 16-29% |
| 12 | 13-23% | 16-29% |
| 13 | 12-22% | 16-29% |
| 14 | 12-21% | 16-30% |
| 15 | 11-21% | 16-30% |
| 16 | 10-20% | 16-30% |
| 17 | 10-20% | 16-30% |
| 18 | 10-20% | 17-31% |
| 19 | 9-20% | 19-32% |
| 20-39 | 8-20% | 21-33% |
| 40-59 | 11-22% | 23-34% |
| 60-79 | 13-25% | 24-36% |

Alternatively, user defined ranges can be defined if preferred. It should be noted that the ranges are independent of the algorithms used in performing the calculation, and just cover what is considered a healthy %Fat Mass for an individual of a given sex and age.

At step 1050 a fat mass indicator is generated using the reference with this being displayed at step 1055.

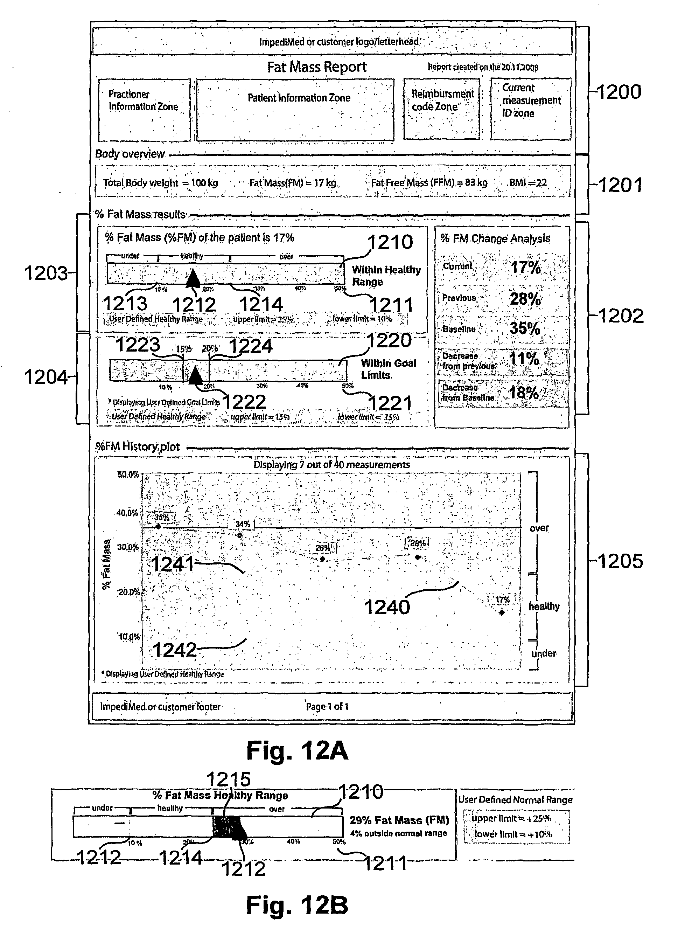

An example of a fat mass report containing a fat mass indicator will now be described with reference to Figures 12A and 12B. In this example the fat mass report includes a header section 1200 similar to the header 1100 described above with respect to the hy-dex report.

The fat mass report includes a summary section 1201, which indicates a summary of the relevant fat mass values including total body weight, fat mass, fat free mass, and body mass index.

First and second indicator sections 1203, 1204 are provided. The first indicator section 1203 includes a linear FM indicator 1210 having an associated scale 1211 and a pointer 1212. In this instance threshold values are shown at 1213 and 1214 respectively, representing the healthy references outlined above. The second indicator section 1204 includes a linear FM indicator 1220 having an associated scale 1221 and a pointer 1222. Threshold values 1223 and 1224 are indicated, which in this instance represent user defined goal limit, allowing a healthcare professional to set targets for subjects.

A progression chart section 1205 includes a line graph 1240 of previous FM results measured for the subject. The graph includes thresholds markings 1241, 1242 corresponding to thresholds either based on the reference population data, or user defined values. It will be appreciated that the history plot can be used to help demonstrate to a subject the effectiveness of any interventions or other treatment regimes such as dieting of the like.

An example of a variation is shown in Figure 12B, in which, if the subject is outside a healthy range, this is indicated by a shaded area shown generally at 1215.

In the event that other body composition indicators are required this can be achieved using steps 1060 to 1080. In this instance at step 1060 the subjects total body water is calculated. This may be achieved in any suitable manner, and may depend on the measurement process performed. However, in one example this is achieved utilising the fat free mass and the following equation:

$$TBW = FFM * 0.721$$

Following this at step 1065 the reactance R and resistance X are used to determine extra cellular and intra cellular water levels for the subject.

In the event that BIA measurements are performed, this is achieved using the following equations:

Females:

$$ECW = \frac{ht^2}{X * 0.012} + \frac{ht^2}{R * 0.053} + wt * 0.095 - 0.64$$

Males:

$$ECW = \frac{ht^2}{X * 0.016} + \frac{ht^2}{R * 0.019} + wt * 0.152 - 3.44$$

The intra-cellular fluid levels are then given by:

$$ICW = TBW - ECW$$

From this, the extracellular mass and active tissue mass can be calculated: Females:

$$ECM = ECW + (ht * 0.531 - 26.83) * 0.12$$

$$ATM = FFM - ECW - (ht * 0.531 - 26.83) * 0.12$$

Males:

$$ECM = ECW + (ht * 0.564 - 29.43) * 0.12$$

$$ATM = FFM - ECW - (ht * 0.564 - 29.43) * 0.12$$

References are then selected at step 1070 with these then being in generating indicators at step 1075, allowing these to be displayed at step 1080.

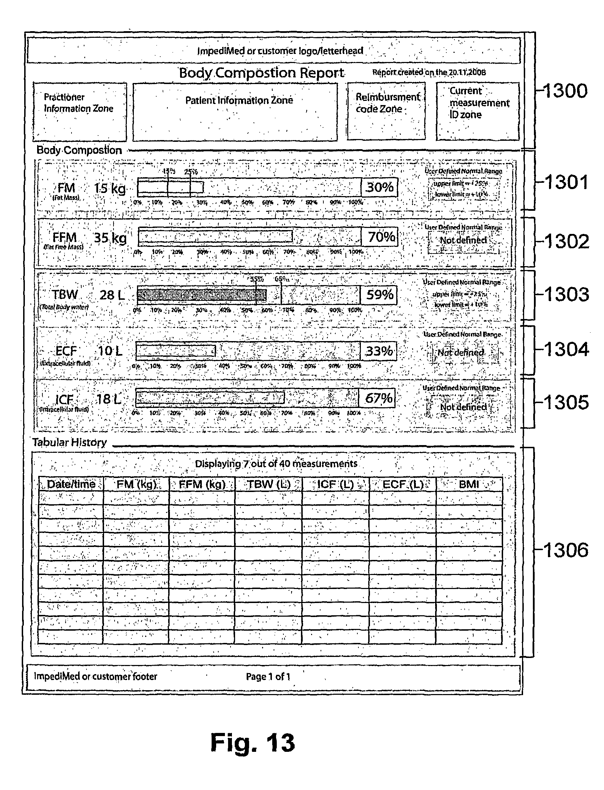

Figure 13 is a body composition report. The body composition report includes a header section 1300 similar to the header sections described above. In addition to this, indicator sections 1301, 1302, 1303,1304, 1305 are provided for displaying indicators of the values of fat mass, fat free mass, total body water, extra cellular fluid and intra cellular fluid respectively. Again each of these indicators includes a linear indicator, associated scale with a relevant pointer, which in this example is in the form of a bar graph. A history section 1306 is also optionally provided, allowing indicator values to be displayed in tabular form.

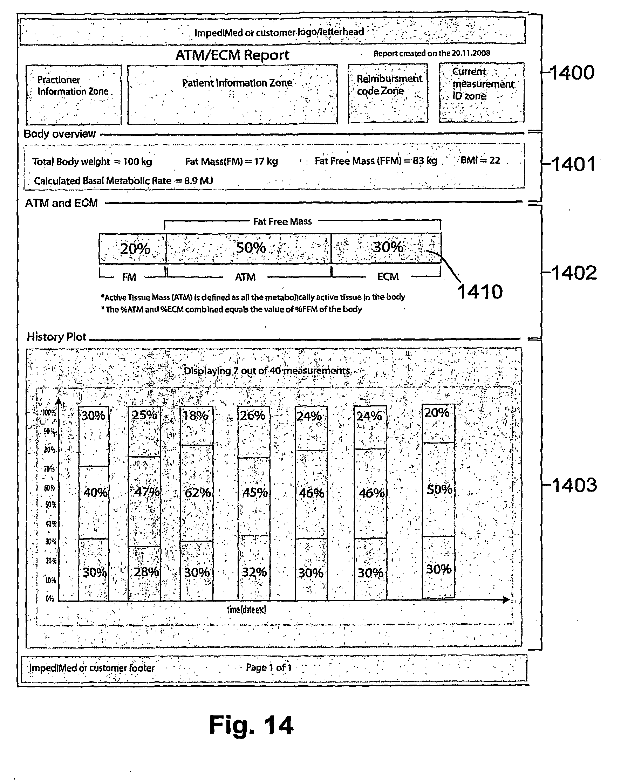

An ATM/ECM report is shown in Figure 14. In this example, the report includes a header section 1400, a summary section 1401, an indicator section 1402 and a history section 1403. The summary section 1401 displays current ATM and ECM values, which are also displayed on a linear indicator 1410 in the indicator section 1402. The history section 1403 includes representations of previously determined ATM and ECM indications. Again the elements are similar to those described above and will not be described in any detail.

Persons skilled in the art will appreciate that numerous variations and modifications will become apparent.

Thus, for example, it will be appreciated that features from different examples above may be used interchangeably where appropriate. Furthermore, whilst the above examples have focussed on a subject such as a human, it will be appreciated that the measuring device and techniques described above can be used with any animal, including but not limited to, primates, livestock, performance animals, such race horses, or the like.

The above described processes can be used for determining the health status of an individual, including the body composition of the individual, or diagnosing the presence, absence or degree of a range of conditions and illnesses, including, but not limited to oedema, lymphoedema, or the like. It will be appreciated from this that whilst the above examples use the term oedema indicator, this is for the purpose of example only and is not intended to be limiting. Accordingly, the oedema indicator can be referred to more generally as an indicator when used in analysing impedance measurements with respect to more general health status information such as body composition, or the like.

**Claims**

1.  A method for use in determining an oedema indicator, the method including, in a processing system:

    a) determining at least one impedance value, representing the impedance of at least a segment of the subject, the at least one impedance value including:

    i) a first measured impedance value representing the impedance of an unaffected limb;
    ii) a second measured impedance value representing the impedance of an affected limb;

    b) determining the oedema indicator indicative of a subject parameter using the at least one impedance value and a reference; and
    c) displaying a representation of the oedema indicator;

    **characterised in that** the oedema indicator is scaled relative to the reference by :

    i) determining an impedance ratio using the first and second measured impedance values

    a. determining a mean impedance ratio value for a normal population;
    b. determining a three standard deviation value for the normal population; and,

    ii) determining the oedema indicator by scaling the impedance ratio using the mean and three standard deviation values using the equation:

$$L - Dex = \frac{sf \times (IR - \mu)}{3\sigma - \mu}$$

    where:

    *L-Dex* is the oedema indicator
    *IR* is the impedance ratio
    $\mu$ is the mean impedance ratio value for a normal population
    $3\sigma$ is the three standard deviation value for the normal population
    *sf* is the scaling factor.

2.  A method according to claim 1, wherein the subject parameter is at least one of:

a) fluid levels within the subject; and,
b) extracellular fluid levels in a subject limb.

3. A method according to claim 1, wherein the method includes, in the processing system, determining the impedance ratio using the equation:

$$IR = \frac{Zul}{Zal}$$

where:

Zul is the measured impedance of the unaffected limb
Zal is the measured impedance of the affected limb

4. A method according to claim 1, wherein the method includes, in the processing system:

a) determining one or more impedance parameter values from measured impedance values, the impedance parameter values include at least one of:

i) an impedance at infinite applied frequency ($R_\infty$);
ii) an impedance at zero applied frequency ($R_0$); and,
iii) an impedance at a characteristic frequency (Zc); and,

b) determining the impedance ratio using the impedance parameter values.

5. A method according to claim 4, wherein the method includes, in the processing system, determining the impedance ratio using the equation:

$$IR = \frac{R_0ul}{R_0al}$$

where:

IR is the impedance ratio
$R_0ul$ is the impedance of the unaffected limb at zero frequency
$R_0al$ is the impedance of the affected limb at zero frequency

6. A method according to claim 1, wherein the scaling factor is at least one of:

a) an integer value.
b) a multiple of ten.

7. A method according to any one of the claims 1 to 6, wherein the reference is at least one of:

a) derived from a normal population; and,
b) determined from predetermined values.

8. A method according to claim 7, wherein the method includes, in the processing system:

a) determining one or more subject details, the subject details include at least one of:

i) limb dominance;
ii) ethnicity;
iii) age;
iv) sex;

v) weight; and,
vi) height; and,

b) selecting the reference at least partially in accordance with the subject details.

9. A method according to any one of the claims 1 to 8, wherein the method includes, in the processing system, displaying the representation as a linear scale, the linear scale including:

a) a linear indicator;
b) a scale; and,
c) a pointer, the pointer being positioned on the scale in accordance with the indicator.

10. A method according to any one of the claims 1 to 9, wherein the method includes, in the processing system, displaying the representation as a linear scale, the linear scale including:

a) a linear indicator;
b) a scale;
c) at least one bar representing the indicator value; and,
d) at least one bar representing at least one of a previous indicator value and a baseline indicator value.

11. A method according to any one of the claims 1 to 10, wherein the method includes, in the processing system:

a) determining at least one threshold using the reference; and,
b) displaying the threshold on the representation, the position of the threshold being indicative of the presence of a condition.

12. A method according to any one of the claims 1 to 11, wherein the method includes, in the processing system, displaying, on the representation, at least one of:

a) a normal range;
b) an intervention range;
c) a hydration range;
d) an oedema range;
e) at least one colour coded region representing a respective range; and,
f) a Gaussian curve representing a normal population distribution.

13. A method according to any one of the claims 1 to 12, wherein method includes in the process system, causing one or more impedance measurements to be performed by:

a) causing at least one excitation signal to be applied to the subject;
b) determining an at least one signal measured across the subject; and,
c) determining at least one impedance value using an indication of the excitation signal and the signal measured across the subject.

14. Apparatus for use in determining an oedema indicator, the apparatus including a processing system for:

a) determining at least one impedance value, representing the impedance of at least a segment of the subject, the at least one impedance value including:

i) a first measured impedance value representing the impedance of an unaffected limb;
ii) a second measured impedance value representing the impedance of an affected limb;

b) determining the oedema indicator indicative of a subject parameter using the at least one impedance value and a reference; and
c) displaying a representation of the oedema indicator;

**characterised in that** the oedema indicator is scaled relative to the reference by:

i) determining an impedance ratio using the first and second measured impedance value; a. determining a mean impedance ratio value for a normal population; b. determining a three standard deviation value for the normal population; and,
ii) determining the oedema indicator by scaling the impedance ratio using the mean and three standard deviation values using the equation:

$$L - Dex = \frac{sf \times (IR - \mu)}{3\sigma - \mu}$$

where:

L-Dex is the oedema indicator
IR is the impedance ratio
$\mu$ is the mean impendance ratio value for a normal population
$3\sigma$ is the three standard deviation value for the normal population
sf is the scaling factor.

**15.** Apparatus according to claim 14, wherein the apparatus includes:

a) a signal generator for applying one or more electrical signals to the subject using a first set of electrodes;
b) a sensor for measuring electrical signals across a second set of electrodes applied to the subject; and,
c) a controller for:

i) controlling the signal generator; and,
ii) determining the indication of the measured electrical signals.

**Patentansprüche**

**1.** Verfahren zur Anwendung beim Ermitteln eines Ödemindikators, wobei das Verfahren in einem Verarbeitungssystem Folgendes beinhaltet:

a) Ermitteln wenigstens eines Impedanzwertes, der die Impedanz von wenigstens einem Segment des Subjekts repräsentiert, wobei der wenigstens eine Impedanzwert Folgendes beinhaltet:

i) einen ersten gemessenen Impedanzwert, der die Impedanz einer unbetroffenen Gliedmaße repräsentiert;
ii) einen zweiten gemessenen Impedanzwert, der die Impedanz einer betroffenen Gliedmaße repräsentiert;

b) Ermitteln des Ödemindikators, der einen Subjektparameter anzeigt, anhand des wenigstens einen Impedanzwertes und einer Referenz; und
c) Anzeigen einer Darstellung des Ödemindikators;
**dadurch gekennzeichnet, dass** der Ödemindikator relativ zu der Referenz skaliert wird durch:

i) Ermitteln eines Impedanzverhältnisses anhand des ersten und zweiten gemessenen Impedanzwertes;

a) Ermitteln eines mittleren Impedanzverhältniswertes für eine Normalbevölkerung;
b) Ermitteln eines Drei-Standard-Abweichungswertes für die Normalbevölkerung; und

ii) Ermitteln des Ödemindikators durch Skalieren des Impedanzverhältnisses anhand des Mittel- und Drei-Standard-Abweichungswertes mit der folgenden Gleichung:

$$L - Dex = \frac{sf \times (IR - \mu)}{3\sigma - \mu}$$

wobei:

> *L-Dex* der Ödemindikator ist;
> IR das Impedanzverhältnis ist;
> $\mu$ der mittlere Impedanzverhältniswert für eine Normalbevölkerung ist;
> $3\sigma$ der Drei-Standard-Abweichungswert für die Normalbevölkerung ist;
> *sf* der Skalierungsfaktor ist.

2. Verfahren nach Anspruch 1, wobei der Subjektparameter wenigstens einer der Folgenden ist:

> a) Fluidspiegel in dem Subjekt; und
> b) extrazelluläre Fluidspiegel in einer Subjektgliedmaße.

3. Verfahren nach Anspruch 1, wobei das Verfahren in dem Verarbeitungssystem das Ermitteln des Impedanzverhältnisses anhand der folgenden Gleichung beinhaltet:

$$IR = \frac{Zul}{Zal}$$

wobei:

> *Zul* die gemessene Impedanz der unbetroffenen Gliedmaße ist;
> *Zal* die gemessene Impedanz der betroffenen Gliedmaße ist.

4. Verfahren nach Anspruch 1, wobei das Verfahren in dem Verarbeitungssystem Folgendes beinhaltet:

> a) Ermitteln von einem oder mehreren Impedanzparameterwerten von gemessenen Impedanzwerten, wobei die Impedanzparameterwerte wenigstens einen der Folgenden beinhalten:
>
>> i) eine Impedanz mit unendlicher angelegter Frequenz ($R\infty$);
>> ii) eine Impedanz mit einer angelegten Null-Frequenz ($R_0$); und
>> iii) eine Impedanz mit einer charakteristischen Frequenz (Zc); und
>
> b) Ermitteln des Impedanzverhältnisses anhand der Impedanzparameterwerte.

5. Verfahren nach Anspruch 4, wobei das Verfahren in dem Verarbeitungssystem das Ermitteln des Impedanzwertes anhand der folgenden Gleichung beinhaltet:

$$IR = \frac{R_0 ul}{R_0 al}$$

wobei:

> *IR* das Impedanzverhältnis ist;
> $R_0 ul$ die Impedanz der unbetroffenen Gliedmaße mit Null-Frequenz ist;
> $R_0 al$ die Impedanz der betroffenen Gliedmaße mit Null-Frequenz ist.

6. Verfahren nach Anspruch 1, wobei der Skalierungsfaktor wenigstens einer der Folgenden ist:

> a) ein ganzzahliger Wert;
> b) ein Vielfaches von zehn.

7. Verfahren nach einem der Ansprüche 1 bis 6, wobei die Referenz wenigstens eines der Folgenden ist:

> a) von einer Normalbevölkerung abgeleitet; und

b) von vorbestimmten Werten ermittelt.

8. Verfahren nach Anspruch 7, wobei das Verfahren in dem Verarbeitungssystem Folgendes beinhaltet:

    a) Ermitteln von einem oder mehreren Subjektdetails, wobei die Subjektdetails wenigstens eines der Folgenden beinhalten:

        i) Gliedmaßendominanz;
        ii) Volkszugehörigkeit;
        iii) Alter;
        iv) Geschlecht;
        v) Gewicht; und
        vi) Größe; und

    b) Auswählen der Referenz wenigstens teilweise gemäß den Subjektdetails.

9. Verfahren nach einem der Ansprüche 1 bis 8, wobei das Verfahren in dem Verarbeitungssystem das Anzeigen der Darstellung als lineare Skala beinhaltet, wobei die lineare Skala Folgendes beinhaltet:

    a) einen linearen Indikator;
    b) eine Skala; und
    c) einen Zeiger, wobei der Zeiger auf der Skala gemäß dem Indikator positioniert ist.

10. Verfahren nach einem der Ansprüche 1 bis 9, wobei das Verfahren in dem Verarbeitungssystem das Anzeigen der Darstellung als lineare Skala beinhaltet, wobei die lineare Skala Folgendes beinhaltet:

    a) einen linearen Indikator;
    b) eine Skala;
    c) wenigstens einen Balken, der den Indikatorwert repräsentiert; und
    d) wenigstens einen Balken, der einen vorherigen Indikatorwert und/oder einen Basislinien-Indikatorwert repräsentiert.

11. Verfahren nach einem der Ansprüche 1 bis 10, wobei das Verfahren in dem Verarbeitungssystem Folgendes beinhaltet:

    a) Ermitteln von wenigstens einem Schwellenwert anhand der Referenz; und
    b) Anzeigen des Schwellenwerts auf der Darstellung, wobei die Position des Schwellenwerts das Vorliegen eines Zustands anzeigt.

12. Verfahren nach einem der Ansprüche 1 bis 11, wobei das Verfahren in dem Verarbeitungssystem das Anzeigen, auf der Darstellung, von wenigstens einem der Folgenden beinhaltet:

    a) einen normalen Bereich;
    b) einen Interventionsbereich;
    c) einen Hydratisierungsbereich;
    d) einen Ödembereich;
    e) wenigstens eine farblich codierte Region, die einen jeweiligen Bereich repräsentiert; und
    f) eine Gaußsche Kurve, die eine Verteilung der Normalbevölkerung repräsentiert.

13. Verfahren nach einem der Ansprüche 1 bis 12, wobei das Verfahren in dem Verarbeitungssystem das Bewirken beinhaltet, dass eine oder mehrere Impedanzmessungen durchgeführt werden durch:

    a) Bewirken, dass wenigstens ein Anregungssignal an das Subjekt angelegt wird;
    b) Ermitteln wenigstens eines über das Subjekt gemessenen Signals; und
    c) Ermitteln wenigstens eines Impedanzwertes anhand einer Anzeige des Anregungssignals und des über das Subjekt gemessenen Signals.

14. Vorrichtung zur Verwendung beim Ermitteln eines Ödemindikators, wobei die Vorrichtung ein Verarbeitungssystem umfasst zum:

a) Ermitteln wenigstens eines Impedanzwertes, der die Impedanz von wenigstens einem Segment des Subjekts repräsentiert, wobei der wenigstens eine Impedanzwert Folgendes beinhaltet:

i) einen ersten gemessenen Impedanzwert, der die Impedanz einer unbetroffenen Gliedmaße repräsentiert;
ii) einen zweiten gemessenen Impedanzwert, der die Impedanz einer betroffenen Gliedmaße repräsentiert;

b) Ermitteln des Ödemindikators, der einen Subjektparameter anzeigt, anhand des wenigstens einen Impedanzwertes und einer Referenz; und
c) Anzeigen einer Darstellung des Ödemindikators;
**dadurch gekennzeichnet, dass** der Ödemindikator relativ zu der Referenz skaliert wird durch:

i) Ermitteln eines Impedanzverhältnisses anhand des ersten und zweiten gemessenen Impedanzwertes;

a) Ermitteln eines mittleren Impedanzverhältniswertes für eine Normalbevölkerung;
b) Ermitteln eines Drei-Standard-Abweichungswertes für die Normalbevölkerung; und

ii) Ermitteln des Ödemindikators durch Skalieren des Impedanzverhältnisses anhand des Mittel- und Drei-Standard-Abweichungswertes mit der folgenden Gleichung:

$$L - Dex = \frac{sf \times (IR - \mu)}{3\sigma - \mu}$$

wobei:

*L-Dex* der Ödemindikator ist;
*IR* das Impedanzverhältnis ist;
$\mu$ der mittlere Impedanzverhältniswert für eine Normalbevölkerung ist;
$3\sigma$ der Drei-Standard-Abweichungswert für die Normalbevölkerung ist;
*sf* der Skalierungsfaktor ist.

**15.** Vorrichtung nach Anspruch 14, wobei die Vorrichtung Folgendes beinhaltet:

a) einen Signalgenerator zum Anlegen von einem oder mehreren elektrischen Signalen an das Subjekt mit einem ersten Satz von Elektroden;
b) einen Sensor zum Messen von elektrischen Signalen über einen zweiten Satz von Elektroden, die auf das Subjekt aufgebracht wurden; und
c) eine Steuerung zum:

i) Steuern des Signalgenerators; und
ii) Ermitteln der Anzeige der gemessenen elektrischen Signale.

**Revendications**

**1.** Procédé servant à déterminer un indicateur d'oedème, le procédé comprenant, dans un système de traitement :

a) l'étape consistant à déterminer au moins une valeur d'impédance, représentant l'impédance d'au moins un segment du sujet, ladite au moins une valeur d'impédance comprenant :

i) une première valeur d'impédance mesurée représentant l'impédance d'un membre non affecté ;
ii) une deuxième valeur d'impédance mesurée représentant l'impédance d'un membre affecté ;

b) l'étape consistant à déterminer l'indicateur d'oedème indiquant un paramètre du sujet en utilisant ladite au moins une valeur d'impédance et une référence ; et

c) l'étape consistant à afficher une représentation de l'indicateur d'oedème ; **caractérisé en ce que** l'indicateur d'oedème est mis à l'échelle par rapport à la référence par les étapes consistant à :

i) déterminer un rapport d'impédance en utilisant les première et deuxième valeurs d'impédance mesurées ;

a. déterminer une valeur moyenne de rapport d'impédance pour une population normale ;
b. déterminer une valeur de trois écarts types pour la population normale ; et,

ii) déterminer l'indicateur d'oedème en mettant à l'échelle le rapport d'impédance en utilisant les valeurs moyenne et de trois écarts types en utilisant l'équation :

$$L - Dex = \frac{sf \times (IR - \mu)}{3\sigma - \mu}$$

où :

*L-Dex* est l'indicateur d'oedème
IR est le rapport d'impédance
$\mu$ est la valeur moyenne de rapport d'impédance pour une population normale
$3\sigma$ est la valeur de trois écarts types pour la population normale
sf est le facteur d'échelle.

2. Procédé selon la revendication 1, dans lequel le paramètre du sujet est au moins l'un parmi :

a) des niveaux de fluide dans le sujet ; et,
b) des niveaux de fluide extracellulaire dans un membre du sujet.

3. Procédé selon la revendication 1, dans lequel le procédé comprend, dans le système de traitement, l'étape consistant à déterminer le rapport d'impédance en utilisant l'équation :

$$IR = \frac{Zul}{Zal}$$

où :

*Zul* est l'impédance mesurée du membre non affecté
*Zal* est l'impédance mesurée du membre affecté.

4. Procédé selon la revendication 1, dans lequel le procédé comprend, dans le système de traitement :

a) l'étape consistant à déterminer une ou plusieurs valeurs de paramètres d'impédance d'après des valeurs d'impédance mesurées, les valeurs de paramètres d'impédance comprenant au moins l'une parmi :

i) une impédance à une fréquence appliquée infinie ($R_\infty$) ;
ii) une impédance à une fréquence appliquée de zéro ($R_0$) ; et,
iii) une impédance à une fréquence caractéristique (Zc) ; et,

b) l'étape consistant à déterminer le rapport d'impédance en utilisant les valeurs de paramètres d'impédance.

5. Procédé selon la revendication 4, dans lequel le procédé comprend, dans le système de traitement, l'étape consistant à déterminer le rapport d'impédance en utilisant l'équation :

$$IR = \frac{R_0ul}{R_0al}$$

où :

IR est le rapport d'impédance

$R_0ul$ est l'impédance du membre non affecté à la fréquence zéro

$R_0al$ est l'impédance du membre affecté à la fréquence zéro.

6. Procédé selon la revendication 1, dans lequel le facteur d'échelle est au moins l'un parmi :

   a) une valeur entière ;
   b) un multiple de dix.

7. Procédé selon l'une quelconque des revendications 1 à 6, dans lequel la référence est au moins l'une parmi :

   a) dérivée à partir d'une population normale ; et
   b) déterminée à partir de valeurs prédéterminées.

8. Procédé selon la revendication 7, dans lequel le procédé comprend, dans le système de traitement :

   a) l'étape consistant à déterminer un ou plusieurs détails du sujet, les détails du sujet comprenant au moins l'un parmi :

      i) la dominance de membre ;
      ii) l'ethnicité ;
      iii) l'âge ;
      iv) le sexe ;
      v) le poids ; et,
      vi) la taille ; et,

   b) l'étape consistant à sélectionner la référence au moins partiellement en fonction des détails du sujet.

9. Procédé selon l'une quelconque des revendications 1 à 8, dans lequel le procédé comprend, dans le système de traitement, l'étape consistant à afficher la représentation sous la forme d'une échelle linéaire, l'échelle linéaire comprenant :

   a) un indicateur linéaire ;
   b) une échelle ; et,
   c) un pointeur, le pointeur étant positionné sur l'échelle en fonction de l'indicateur.

10. Procédé selon l'une quelconque des revendications 1 à 9, dans lequel le procédé comprend, dans le système de traitement, l'étape consistant à afficher la représentation sous la forme d'une échelle linéaire, l'échelle linéaire comprenant :

    a) un indicateur linéaire ;
    b) une échelle ;
    c) au moins une barre représentant la valeur de l'indicateur ; et,
    d) au moins une barre représentant au moins l'une parmi une valeur d'indicateur précédente et une valeur d'indicateur de base.

11. Procédé selon l'une quelconque des revendications 1 à 10, dans lequel le procédé comprend, dans le système de traitement :

    a) l'étape consistant à déterminer au moins un seuil en utilisant la référence ; et,
    b) l'étape consistant à afficher le seuil sur la représentation, la position du seuil indiquant la présence d'une condition.

**12.** Procédé selon l'une quelconque des revendications 1 à 11, dans lequel le procédé comprend, dans le système de traitement, l'étape consistant à afficher, sur la représentation, au moins l'un parmi :

a) un niveau normal ;
b) un niveau d'intervention ;
c) un niveau d'hydratation ;
d) un niveau d'oedème ;
e) au moins une région à code de couleur représentant un niveau respectif ; et,
f) une courbe gaussienne représentant une distribution de population normale.

**13.** Procédé selon l'une quelconque des revendications 1 à 12, dans lequel le procédé comprend, dans le système de traitement, l'étape consistant à amener une ou plusieurs mesures d'impédance à être effectuées par les étapes consistant à :

a) amener au moins un signal d'excitation à être appliqué sur le sujet ;
b) déterminer un au moins un signal mesuré en travers du sujet ; et,
c) déterminer au moins une valeur d'impédance en utilisant une indication du signal d'excitation et le signal mesuré en travers du sujet.

**14.** Appareil servant à déterminer un indicateur d'oedème, l'appareil comprenant un système de traitement servant à :

a) déterminer au moins une valeur d'impédance, représentant l'impédance d'au moins un segment du sujet, ladite au moins une valeur d'impédance comprenant :

i) une première valeur d'impédance mesurée représentant l'impédance d'un membre non affecté ;
ii) une deuxième valeur d'impédance mesurée représentant l'impédance d'un membre affecté ;

b) déterminer l'indicateur d'oedème indiquant un paramètre du sujet en utilisant ladite au moins une valeur d'impédance et une référence ; et
c) afficher une représentation de l'indicateur d'oedème ;

**caractérisé en ce que** l'indicateur d'oedème est mis à l'échelle par rapport à la référence par les étapes consistant à :

i) déterminer un rapport d'impédance en utilisant les première et deuxième valeurs d'impédance mesurées ;

a. déterminer une valeur moyenne de rapport d'impédance pour une population normale ;
b. déterminer une valeur de trois écarts types pour la population normale ; et,

ii) déterminer l'indicateur d'oedème en mettant à l'échelle le rapport d'impédance en utilisant les valeurs moyenne et de trois écarts types en utilisant l'équation :

$$L - Dex = \frac{sf \times (IR - \mu)}{3\sigma - \mu}$$

où :

*L-Dex* est l'indicateur d'oedème
*IR* est le rapport d'impédance
$\mu$ est la valeur moyenne de rapport d'impédance pour une population normale
$3\sigma$ est la valeur de trois écarts types pour la population normale
*sf* est le facteur d'échelle.

**15.** Appareil selon la revendication 14, dans lequel l'appareil comprend :

a) un générateur de signaux servant à appliquer un ou plusieurs signaux électriques sur le sujet en utilisant un premier ensemble d'électrodes ;

b) un capteur servant à mesurer des signaux électriques en travers d'un deuxième ensemble d'électrodes appliquées sur le sujet ; et

c) un dispositif de commande servant à :

   i) commander le générateur de signaux ; et,

   ii) déterminer l'indication des signaux électriques mesurés.

**Fig. 1**

Apply current
signal to subject S    200

Measure voltage
signals across        210
subject S

Process voltage and
current signals to
determine             220
impedance value(s)

Use impedance
values and reference   230
to determine
indicator

Optionally display     240
indicator

# Fig. 2

**Fig. 3A**

**Fig. 3B**

**Fig. 3C**

```
┌─────────────────┐
│ Determine subject │        400
│     details       │
└─────────────────┘
         │
         ▼
┌─────────────────┐
│    Determine      │        410
│  affected limb    │
└─────────────────┘
         │
         ▼
┌─────────────────┐
│ Position impedance│
│ electrodes on the │        420
│    subject S      │
└─────────────────┘
         │
         ▼
┌─────────────────┐
│    Determine      │
│    impedance      │        430
│  measurements     │
└─────────────────┘
         │
         ▼
┌─────────────────┐
│ Optionally derive │
│    impedance      │        440
│   parameters      │
└─────────────────┘
         │
         ▼
┌─────────────────┐
│ Determine a limb  │        450
│ impedance ratio IR│
└─────────────────┘
         │
         ▼
┌─────────────────┐
│                   │
│ Select reference  │        460
│                   │
└─────────────────┘
         │
         ▼
┌─────────────────┐
│  Calculate limb   │
│ oedema indicator  │        470
│  using reference  │
└─────────────────┘
         │
         ▼
┌─────────────────┐
│     Display       │
│ representation of │        480
│ oedema indicator  │
│      value        │
└─────────────────┘
```

# Fig. 4

**Fig. 5A**

**Fig. 5B**

**Fig. 5C**

**Fig. 5D**

600

311    320         302    312

L-Dex normal range
mean

-10.0          0.0          10.0          20.0

300

**Fig. 6A**

Date 12/12/2006

L-Dex:    7.0

In normal range

601

---

600

311    320         312    302

L-Dex normal range
mean

-10.0          0.0          10.0          20.0

300

**Fig. 6B**

Date 12/12/2006

L-Dex:  12.0

Out of normal range

601

---

600

311    320         302    312    300

L-Dex normal range
mean

-10.0          0.0          10.0          20.0

320

**Fig. 6C**

Date 12/12/2006

L-Dex:  - 12.0

Investigation of result
Required

601

**Fig. 6D**

**Fig. 7A**

**Fig. 7B**

**Fig. 8A**

**Fig. 8B**

**Fig. 8C**

101 Nomination Drive
Capital City
State 1234

**Nominated Health Centre**

**"Sorting you out"**

Appointments 01 234 5678
General office 01 345 6789
Fax 01234 5789
enquiries@nom.health.org

180 by 25 mm user logo graphic for first report page

**LYMPHOEDEMA ANALYSIS REPORT**          CPT Code          ⌐900

**Patient details**

| | | | |
|---|---|---|---|
| Name: | Miranda P Sykes | | |
| Date of birth: | 30/7/1931 | Dominant: | Right |
| PID: | H 214-03-087 | Affected: | Left |

| | |
|---|---|
| Practitioner: | Dr Fred P Howlett |
| Report printed at: | 11/1/2007 12:34:56 |
| Sample ID | 11043-0267 |

**Current analysis**

L-Dex normal range
mean
-10.0   0.00   10.0   20.0

Date 12/12/2006

L-Dex: 7.0

✓ In normal range          ⌐901

*L-Dex values greater than 10 are indicative of lymphoedema. The L-Dex scale is a tool to help in the diagnostic and monitoring process of lymphoedema but is not a definitive diagnostic tool.*

**Change analysis**

L-Dex normal range
mean

CURRENT 7.0          12/12/2006
PREVIOUS 12.0          31/10/2006
BASELINE -1.0
-10.0   0.00   10.0   20.0

↘ Decrease from previous 5.0

↗ Increase from baseline 8.0          ⌐902

**History**

20.0
10.0                    1.073
                   1.047          1.034
          0.992
L-Dex   0.958
0.00

-10.0   Surgery

16/3/2006   24/4/2006   19/7/2006   31/10/2006   12/12/2006
12:34:56   18:54:13   11:39:05   14:28:25   10:39:05

Mean
Baseline          ⌐903

**Report notes**

Practitioner's typed notes here.
If there is not enough room in this box, the notes will spill onto continuation pages.
The signature box below is at the end of the notes, however many pages they take up.          ⌐904

| SIGNED | DATE |
|---|---|

**Fig. 9**

```
                    ┌─────────────────┐
                    │ Determine subject │    1000
                    │     details      │
                    └─────────────────┘
                            │
                            ▼
                    ┌─────────────────┐
                    │ Position impedance │  1005
                    │ electrodes on the │
                    │    subject S     │
                    └─────────────────┘
                            │
                            ▼
                    ┌─────────────────┐
                    │    Determine    │    1010
                    │    impedance    │
                    │  measurements   │
                    └─────────────────┘
                            │
                            ▼
                    ┌─────────────────┐
                    │ Derive impedance │   1015
                    │   parameters    │
                    └─────────────────┘
```

|  |  |  |  |  |  |
|---|---|---|---|---|---|
| 1020 | Normalised resistance R and reactance Xc values | 1040 | Use relevant FFM equation to determine FFM and FM | | Calculate total body water | 1060 |
| 1025 | Select reference | 1045 | Select reference | | Use resistance and reactance to determine ECW and ICW | 1065 |
| 1030 | Calculate hy-dex indicator using reference | 1050 | Generate FM indicator using reference | | Select references | 1070 |
| 1035 | Display representation of hy-dex indicator value | 1055 | Display representation of FM indicator value | | Calculate body composition indicators using references | 1075 |
| | | | | | Display representation of body composition indicators | 1080 |

# Fig. 10A

**Fig. 10B**

**Fig. 10C**

**Fig. 10D**

Fig. 11A

Fig. 11B

Fig. 11C

ImpediMed or customer logo/letterhead

## Fat Mass Report

Report created on the 20.11.2008

| Practioner Information Zone | Patient Information Zone | Reimbursment code Zone | Current measurement ID zone |

—1200

Body overview

Total Body weight = 100 kg    Fat Mass(FM) = 17 kg    Fat Free Mass (FFM) = 83 kg    BMI = 22

—1201

% Fat Mass results

% Fat Mass (%FM) of the patient is 17%    **1210**

under — healthy — over

Within Healthy Range

10%   20%   30%   40%   50%

**1213**   **1212**   **1214**    **1211**

User Defined Healthy Range   upper limit = 25%   lower limit = 10%

**1223**   15%   20%   **1224**    **1220**

Within Goal Limits

10%   20%   30%   40%   50%

Displaying User Defined Goal Limits   **1222**

User Defined Healthy Range   upper limit = 15%   lower limit = 15%    **1221**

1203 — 1204

% FM Change Analysis

| Current | 17% |
| Previous | 28% |
| Baseline | 35% |
| Decrease from previous | 11% |
| Decrease from Baseline | 18% |

—1202

%FM History plot

Displaying 7 out of 40 measurements

50.0%

40.0%   35%   34%

30.0%   28%   28%   over

% Fat Mass

**1241**

20.0%

**1240**   17%   healthy

10.0%

**1242**   under

* Displaying User Defined Healthy Range

—1205

ImpediMed or customer footer    Page 1 of 1

## Fig. 12A

**1215**

% Fat Mass Healthy Range    **1210**

under — healthy — over

29% Fat Mass (FM)
4% outside normal range

10%   20%   30%   40%   50%

**1212**   **1214**   **1212**    **1211**

User Defined Normal Range

upper limit = +25%
lower limit = +10%

## Fig. 12B

ImpediMed or customer logo/letterhead

## Body Compostion Report
Report created on the 20.11.2008 — 1300

| Practioner Information Zone | Patient Information Zone | Reimbursment code Zone | Current measurement ID zone |

**Body Compostion**

FM 15 kg (Fat Mass) — 15% 25% — 0% 10% 20% 30% 40% 50% 60% 70% 80% 90% 100% — 30% — User Defined Normal Range upper limit = 25% lower limit = 10% — 1301

FFM 35 kg (Fat Free Mass) — 0% 10% 20% 30% 40% 50% 60% 70% 80% 90% 100% — 70% — User Defined Normal Range Not defined — 1302

TBW 28 L (Total Body water) — 55% 65% — 0% 10% 20% 30% 40% 50% 60% 70% 80% 90% 100% — 59% — User Defined Normal Range upper limit = 25% lower limit = 10% — 1303

ECF 10 L (Extracellular fluid) — 0% 10% 20% 30% 40% 50% 60% 70% 80% 90% 100% — 33% — User Defined Normal Range Not defined — 1304

ICF 18 L (Intracellular fluid) — 0% 10% 20% 30% 40% 50% 60% 70% 80% 90% 100% — 67% — User Defined Normal Range Not defined — 1305

**Tabular History**

Displaying 7 out of 40 measurements

| Date/time | FM (kg) | FFM (kg) | TBW (L) | ICF (L) | ECF (L) | BMI |
|---|---|---|---|---|---|---|
| | | | | | | |
| | | | | | | |
| | | | | | | |
| | | | | | | |
| | | | | | | |
| | | | | | | |
| | | | | | | |
| | | | | | | |
| | | | | | | |
| | | | | | | |

— 1306

ImpediMed or customer footer          Page 1 of 1

## Fig. 13

Fig. 14

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0079255 A
- WO 2007041783 A
- US 20040167423 A
- US 20060224079 A

**Non-patent literature cited in the description**

- **PICCOLI et al.** Impedance vector distribution by sex, race, body mass index and age in the United States: standard reference intervals as bivariate Z scores. *Nutrition,* 2002, vol. 18, 153-167